(19) Europäisches Patentamt
European Patent Office
Office européen des brevets

(11) **EP 2 624 910 B1**

(12) **EUROPEAN PATENT SPECIFICATION**

(45) Date of publication and mention
of the grant of the patent:
**02.09.2020 Bulletin 2020/36**

(21) Application number: **11831732.0**

(22) Date of filing: **07.10.2011**

(51) Int Cl.:
*A61N 2/02* (2006.01)         *A61N 5/00* (2006.01)
*A61B 18/18* (2006.01)        *A61B 5/02* (2006.01)
*A61H 39/00* (2006.01)

(86) International application number:
**PCT/US2011/055487**

(87) International publication number:
**WO 2012/048302 (12.04.2012 Gazette 2012/15)**

(54) **HANDHELD EXCITATION TERMINAL AND EMF EMITTER PROVIDING DYNAMIC OPTIMIZATION OF EMISSION AND THERAPEUTIC EFFECT**

TRAGBARER ERREGUNGSANSCHLUSS UND EMF-SENDER ZUR DYNAMISCHEN OPTIMIERUNG VON EMISSIONS- UND THERAPIEWIRKUNGEN

TERMINAL D'EXCITATION PORTATIF ET ÉMETTEUR EMF PERMETTANT L'OPTIMISATION DYNAMIQUE DE L'ÉMISSION ET DE L'EFFET THÉRAPEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2011 US 201113226245**
**07.10.2010 US 391037 P**

(43) Date of publication of application:
**14.08.2013 Bulletin 2013/33**

(73) Proprietor: **Biomobie Corp.**
**Cupertino, California 95104 (US)**

(72) Inventors:
• **WANG, Jian**
**Cupertino, California 95104 (US)**

• **KU, Edmond**
**Cupertino, California 95104 (US)**

(74) Representative: **Schweiger, Martin**
**Schweiger & Partners**
**Intellectual Property Law Firm**
**Elsenheimer Strasse 1**
**80687 München (DE)**

(56) References cited:
**WO-A1-96/32158          WO-A1-2005/084753**
**WO-A1-2010/006175     WO-A1-2010/025114**
**US-A- 5 458 142           US-A1- 2003 130 709**
**US-A1- 2005 288 744    US-A1- 2009 030 476**
**US-A1- 2009 240 310    US-A1- 2010 004 551**
**US-B1- 7 801 585**

**Description**

**Field of the Invention**

The present invention relates to an electronic handheld cell excitation terminal adapted to emit electromagnetic waves that is capable of dynamic optimization of a therapeutic effect.

**BACKGROUND**

Traditional needle-based acupuncture therapeutic techniques has been practiced for many years. For acupuncture therapy, needles are inserted into or close to meridian points along the patient's body to effect the desired and beneficial therapeutic results.

Conventionally, therapeutic devices and equipment are available where the system completely bathes the patient within the electromagnetic field. By enveloping the patient in the field, the system attempts to achieve the same desired and beneficial therapeutic effect as traditional acupuncture techniques.

The electromagnetic field from these traditional devices, systems, and equipment are non-selective, and non-focused thus the efficacy of this type of system is questionable. Moreover, due to the generic nature of the platform nothing can be customized and optimized to address the patient's condition, so the therapeutic effects and benefits of such platforms cannot be guaranteed and time spent on such therapy may not be useful.

A different class of broadband electromagnetic health devices utilizes visible red light and infrared wavelengths (i.e., a much higher portion of the electromagnetic spectrum) as the therapeutic mechanism. This class of devices claims to have the ability to improve the user's immune system and blood circulation.

Other systems utilize a static magnetic field (i.e., those generated by magnets) and through different motion mechanisms generate a cyclical moving field. Moreover, a significant drawback of these systems is that they are mechanical in nature and thus cannot provide pulse electromagnetic fields, which have been found to provide the best efficacy. Although the device can be designed to provide different field strengths and frequencies, again due to the mechanical nature of the field generation it is not possible for these platforms to be customized and optimized for each patient to achieve the optimal benefits.

The WO 2005/084753 discloses a hand held device with a noncontact thermopile for detecting infrared radiation from a human or animal body, means for detecting electromagnetic characteristics exhibited by the body and PEMFT means for generating pulsed magnetic wave forms.

The US 2010/0004551 discloses a device for applying modulated magnetic fields and/or modulated light to a body. The device comprises sensors for recording ECG signals and pulse waves.

The WO 2010/025114 discloses a system and method for providing a magnetic resonance treatment. In one embodiment, sensors, including, for example, biometric sensors are in communication with the subject.

The WO 2010/006175 discloses an apparatus for providing and using a driver for a magnetic field. A sensor is configured to monitor physical parameters of a subject receiving the magnetic field, such as an EGK, an EEG, a heart rate monitor, or a blood pressure monitor.

The US 7,801,585 discloses a system for analyzing and treating abnormality of human and animal tissues with a subsystem for modulating a magnetic field.

The US 5,458,142 discloses a diagnostic and therapeutic instrument for use in the treatment of living organisms with a sensor for detecting magnetic fields emanating from the living organism.

SUMMARY

The invention is defined by the claims. The present invention is directed to an electronic therapeutic device and system using customized emitted electromagnetic (EM) waves varying dynamically with time for excitation, which uses electromagnetic waves having biologic cell or tissue efficacy (bioelectromagnetic waves) corresponding to expected physical status of a receiver or patient body to perform therapeutic radiation on a specific part of the receiver or patient's body. It also advantageously but optionally but advantageously provides diagnostic information relating to a biologic tissue or patient status before, during, and/or following treatment. In one particular example, the present invention provides a small portable handheld electronic therapeutic device that is held in palm of the patient undergoing treatment and that provides the EMW emitter, sensors, and wireless communication interface for communication with external system elements.

The present invention relates to a portable and/or handheld cell or biological tissue excitation terminal electronic device for generating and emitting an electromagnetic field having particular electromagnetic (EM) wave and electronic characteristics. The use of the device for application of the EM wave or waves to biological tissue, cells, or animal or human for diagnostic and/or therapeutic effects, particularly in conjunction with a remote system server that provides data storage and optional data or information processing through communications between the portable electronic device and the remote system server via a communications network. The cell or tissue excitation terminal can perform direct therapy on a human body, and can be used in combination with other devices of the system through a wired and/or wireless network, so as to perform useful therapy on a human body while utilizing remotely stored data and control information.

In another aspect, the innovation provides a portable device for emitting electromagnetic pulses and streaming the electromagnetic pulses continuously to a defined and limited region of the human body - specifically the center of the user's palm or sole of the foot. The electromagnetic pulse shapes and waveform characteristics are dynamically configurable and selectable through software during the treatment and/or from treatment to treatment over a multi-session course of treatment or therapy.

In another aspect, the innovation provides a heartbeat rate sensing system including: an accelerometer for sensing a movement of a body part caused by blood flow or heartbeat vibrations, or an infrared reflective sensor that senses blood flow through the dermis; a digital signal processor for extracting the heartbeat or pulse beat from sensed signals; and processing logic for determining the heartbeat or pulse rate and the time of the next heartbeat or pulse.

In another aspect, the innovation provides a portable device for emitting electromagnetic pulses and delivering a stream of electromagnetic pulses and altering the magnetic field to a defined and limited region of the human body - specifically the center of the user's palm or sole of the foot, where the electromagnetic pulses having wave shapes and waveform characteristics that are dynamically configurable and selectable by software during the treatment, and wherein a heartbeat rate is sensed by the device and a modified and/or modulated electromagnetic wave is delivered taking into account the heartbeat rate and/or heartbeat timing or according to other sensed patient conditions. Delivering the stream of electromagnetic pulses near the center of the user's palm or sole of the foot is also effective but perhaps not as effective as at the center.

In another aspect, the innovation provides a system including: a plurality of portable devices for emitting electromagnetic wave signals or pulses and delivering separate streams of electromagnetic pulses, altering the electromagnetic, electric, and/or magnetic fields, to separate defined and limited regions of the human body; each of the electromagnetic pulses optionally but desirable in having wave shapes and waveform characteristics that are dynamically configurable and selectable during the treatment through software, and being emitted and delivered from each device to a particular body part. In some examples of the device, system, and method, the body part is a body part at or near a meridian point. In another example, the device and method are applied to the palm of the hand, while in another example, they are applied to the sole of the foot; each of these later examples having demonstrated particular beneficial therapeutic effect.

The portable devices emitting a treatment dose of electromagnetic radiation to a limited area of the tissue or body and not to the entire human body. For example, in one example, the emitter emits the electromagnetic field to an area between 1.27 - 5.08 cm (a ½ inch to 2 inches) in diameter, and more typically approximately 2.54 cm (one-inch) in diameter, for example in one non-limiting example between substantially 1.78 cm (0.7 inches) and 3.3 cm (1.3 inches) in diameter. While circular emission patters are effective (e.g. diameter, it will be appreciated that neither the coil nor the emission patter needs to be circular and that the coils may be shaped to provide oval, annular, or other pattern. Each portable device includes a wireless communication interface, providing a wireless two-way communication connection to a network capable of intermittent connection to network servers, which provide storage and processing for patient specific waveforms, patient treatment data, and patient specific settings for the portable device.

In another aspect, the innovation provides a portable device as above, wherein the device is powered from an internal rechargeable battery and has physical dimensions less than 10.16 cm by 10.16 cm by 5.08 cm (4 inches by 4 inches by 2 inches).

In another aspect, the innovation provides portable device as above, wherein the device emits an electromagnetic field strength of between 10 uT and 500 uT.

In another aspect, the innovation provides a portable device as above, wherein the wireless interface comprises at least one of the following: a Bluetooth wireless interface, a WiFi wireless interface, and a cellular telephone wireless interface.

The disclosure provides a method of applying an electromagnetic wave to tissue, including: during a first period of time, applying a pulsed electromagnetic field having a first electromagnetic field characteristic; and during a second period of time, applying a different pulsed electromagnetic field having a second electromagnetic field characteristic different from the first electromagnetic field characteristic, where the second period of time corresponding to the time of a heartbeat or heartbeat pulse; and dynamically modifying the first electromagnetic field characteristic and the second electromagnetic field characteristic in response to feedback from the heartbeat sensor.

The disclosure provides a method of diagnosing a condition of a body using electromagnetic radiation to a meridian line or point of a human body, and sensing the reaction of the applied radiation to determine a condition of the patient and to determine and identify, adapt, or modify a treatment regime.

The present invention is directed to a therapeutic system using customized EM waves varying dynamically with time for excitation, which uses bioelectromagnetic waves corresponding to expected physical status of a receiver to perform radiation on a specific part of the receiver. It also advantageously but optionally provides diagnostic information relating to a biologic tissue or patient status before, during, and/or following treatment.

In order to achieve the objective, one example of the present invention provides a portable or handheld cell excitation terminal or device adapted for and capable of dynamic optimization of a therapeutic effect.

In one non-limiting example, the portable or handheld cell excitation terminal or device includes: a processor, processing logic circuit, or central processing unit (CPU); a human body status detection device or sensor connected to the processor or CPU and used to detect human body status information; one or more Electromagnetic (EM) wave generators, where each of the EM wave generators is connected to the processor or CPU, and the processor or CPU controls, according to a signal detected by the human body status detection device, the bioelectromagnetic wave generator adapted and configured to emit or send bioelectromagnetic waves corresponding to a detected status or subject; and a power device, used to supply power to the devices.

In one non-limiting example, the power device is or includes an electrochemical based or standard battery, such as conventional alkaline, NiCad, nickel metal hydride, or Lithium Ion based battery. Advantageously, the batteries are rechargeable and the device 5 may advantageously either include recharging circuits or be coupleable to a recharging circuit through an external charger. The power supply may also optionally or alternatively provide the supply power to power the device using high-capacity super capacitors.

The present invention may advantageously further provide or interoperate with a remote medical system. In one non-limiting example of this a remote medical system, it includes: a control integrated circuit, processor., processing logic circuitry, or a central processing unit (CPU); a cell or biological tissue excitation terminal or device, where the cell or biological tissue excitation terminal or device includes: a biological tissue or human body status detection device or sensor, used to sense or detect biological tissue human body status information; a communication device or circuit for communication between the cell excitation terminal and with the outside such as with the remote medical system or an intermediate unit of system interposed between the excitation terminal and the remote medical system; one or more bioelectromagnetic wave generators, where each of the EM wave generators is connected through driving electronics to the processor or CPU, and the processor or CPU controls, according to a signal detected by the human body status detection device or sensor, the bioelectromagnetic wave generator adapted and configured to send bioelectromagnetic waves corresponding to a detected status or subject; and a power device, used to supply power such as a voltage and/or a current to the devices; a computer or server, where the computer server is connected to the cell or tissue excitation terminal or device through a wired or wireless network, and receives and processes the human body status detection information sent by the cell or tissue excitation terminal, and sends an instruction or other information, so that the processor or CPU of the cell or tissue excitation terminal controls or otherwise influences the operation of the bioelectromagnetic wave generator to send the bioelectromagnetic waves corresponding to the detected status or subject.

Advantageously, a wireless network or communication link between the cell or tissue excitation terminal or device and the computer or server is used so as not to encumber with wires application of the EM waves to the patient body part. It will be understood that the cell or tissue excitation terminal or device may communicate in a wireless manner to an intermediate wireless communication transmitter/receiver (transceiver) and that the intermediate wireless communication device may then connect to the external server. Various wireless communication means and protocols as are known in the art may be used, such as but not limited to various cellular telephone signals and protocols, WiFi, WiMax, Bluetooth, other proprietary wireless signaling means and protocols, and others as are known in the art or that may be developed in the future.

Furthermore, the remote medical server may optionally but advantageously include or may be coupled or connected with a database, used to store the detected human body status information, other associated therapeutic information, various therapeutic waveforms or waveform definitions that may be used to generate the actual therapeutic waveforms, and a mapping relationship list, table, or data structure of patient sickness or conditions and waveforms, the mapping relationship list, table, or data structure listing or otherwise providing therapeutic bioelectromagnetic waves or bioelectromagnetic wave combinations and therapeutic means corresponding to different human body status.

Furthermore, the server further optionally but advantageously includes an optimization module, adapted and configured for updating the mapping relationship list, table, or data structure of patient sickness or condition and waveforms from time to time, so as to update the relationship list with optimal or recommended therapeutic bioelectromagnetic waves.

In accordance with the inventive system, the EMF is a pulsed EMF (PEMF).

The cell or tissue excitation terminal 5 may optionally but advantageously include a power monitoring device, sending out a signal to call attention to power supplement or recharge, when the battery power is too low to sustain performance of the terminal.. For example, there may be an audible sound or beep or an indicator light or an icon on the display on the portable device when remaining power is low. Alternatively or in addition, the signal may be communicated to an external device or system to inform that system or an operator or therapist associated with that system that power is low.

Advantageously, the system and/or device will include a prediction logic and/or software or algorithm operating in conjunction with the device 5 or overall system to predict the total power required for the particular patient treatment session so that it will be apparent before the treatment session commences that there is sufficient power to complete the predicted treatment, even advantageously taking into account how the therapeutic treatment may be dynamically and adaptively be modified.

In one non-limiting example, an additional power supply may be attached to the portable device via a plug-in or other coupling terminal so that treatment may with certainty have sufficient operating power. Where treatment may be extended, an external power supply may be used exclusively for operating the portable device 5 so that any internal battery may be primarily used to operate the logic circuits, sensors, and/or communications or some subset of these, and the power to generate and emit the electromagnetic, electric, and/ or magnetic fields or waves provided by the external power supply. Although this may somewhat encumber the attachment of the device 5 to the patient's body, as it is only a thin wired cord (for example, two-wire low voltage electrical cord) that may be connected to one or each portable device after placement, the encumbrance is not significant.

Furthermore, the bioelectromagnetic wave generator may optionally but advantageously include a waveform multiplexer, adapted and configured for selecting corresponding bioelectromagnetic waves stored locally in the terminal or from those stored in or generated by a server for processing and outputting one or several required or desirable bioelectromagnetic waves, these several waves may be the same or have different characteristics, where in at least some examples the several or plurality of waves have different characteristics.

In accordance with the invention the human body status detection device includes a heartbeat detector.

In accordance with one non-limiting example, the human body status detection device further includes or may be coupled for communication with a blood pressure detector.

In accordance with one non-limiting example, the human body status detection device further includes or may be coupled for communication with a motion sensor.

In accordance with one non-limiting example, the human body status detection device optionally but advantageously may further include or may be coupled with a blood oxygenation level sensor.

Furthermore, the bioelectromagnetic wave generator optionally but advantageously includes a drive circuit, receiving a waveform signal from a waveform generator or a waveform shape generator and driving an EM circuit to generate corresponding electromagnetic waves for application to a biological tissue (referred to as bioelectromagnetic waves).

The cell or tissue excitation terminal or device optionally but advantageously includes a digital-analog (D/A) converter, receiving a digital signal from the processor or CPU and outputs an analog signal to the waveform shaper generator.

In at least one non-limiting example, the heartbeat and/or pulse detection sensors are included within the portable device since they may sense the heartbeat and/or pulse where the portable device is applied or in contact with the tissue or skin of the patient's body. Where a status cannot be determined at the location of the portable device, such as a blood oxygenation level that may require a sensor that can detect tissue color or transmission though a portion of a patient's body (e.g., a person's fingertip) then the portable device may receive directly or indirectly such sensor information.

In one aspect therefore, the present invention provides a portable therapeutic system using customized bioelectromagnetic waves varying dynamically with time for excitation, and through the sensing of real-time or near real-time physical status information of a patient, for example a heartbeat or pulse frequency status, perform real-time optimizations of the waveform by using a different or modified Electromagnetic (EM) pulse or EM pulse combination thereof, so as to achieve a desired effect and thereby further improving the therapeutic effect. Furthermore, the therapeutic system, device, and method according to examples can be managed remotely. Remote management of the device may advantageously include a remote server that optimizes and updates therapeutic waveforms for a patient constantly, or according to some plan, or based on sensory feed-back or status information according to a therapeutic effect on the patient, so as to be adapted for different patients or different physical status of a patient in different time, thereby improving the therapeutic effect dynamically and adaptively constantly or if not constantly according to a treatment plan or other criteria.

Other and different aspects will become apparent from the description provided throughout this document and from the drawings.

**BRIEF DESCRIPTION OF THE DRAWINGS**

FIG. 1 is a schematic view of a remote diagnostic and therapeutic system using customized EM waves varying dynamically with time for excitation;

FIG. 2 is a schematic view of an embodiment of a cell excitation terminal of the remote therapeutic system using customized EM waves varying dynamically with time for excitation according to the present invention;

FIG. 3 illustrates a pulse width modulation (PWM) waveform with a duty factor (black) varying with time;

FIG. 4 is a flow chart of operation of a central control unit;

FIG. 5 is an embodiment of an optimization process of a mapping relationship list of patient sickness and waveforms;

FIG. 6 is an embodiment of a mapping relationship list of patient sickness and waveforms according to the present invention;

FIG. 7 is a view of a waveform of an EM wave having preferred therapeutic effect;

FIG. 8 is a measurement table of therapeutic effects of the waveform in FIG. 7 in respect of various sickness;

FIG. 9A and FIG. 9B are comparison diagrams of effects on blood before and after a therapeutic scheme according to the present invention is implemented; and

FIG. 10A and FIG. 10B are comparison diagrams of measured effects after a therapeutic scheme is applied on a specific treated part for a certain period of time, where FIG. 10A-1 is a before treatment diagram, FIG 10A-2 is an after treatment diagram, FIG. 10B-1 is a before treatment diagram, and FIG. 10B-2 is an after treatment diagram.

FIG. 11 is a diagram showing a further example of the cell or tissue excitation device functional components.

FIG. 12 is a diagram showing a additional detail of the functional components of an embodiment of the cell or tissue excitation device.

FIG. 13 is a diagram showing an example of the form and shape of the cell or tissue excitation unit.

## DETAILED DESCRIPTION

Embodiments of the present invention are described below in detail with reference to the accompanying drawings.

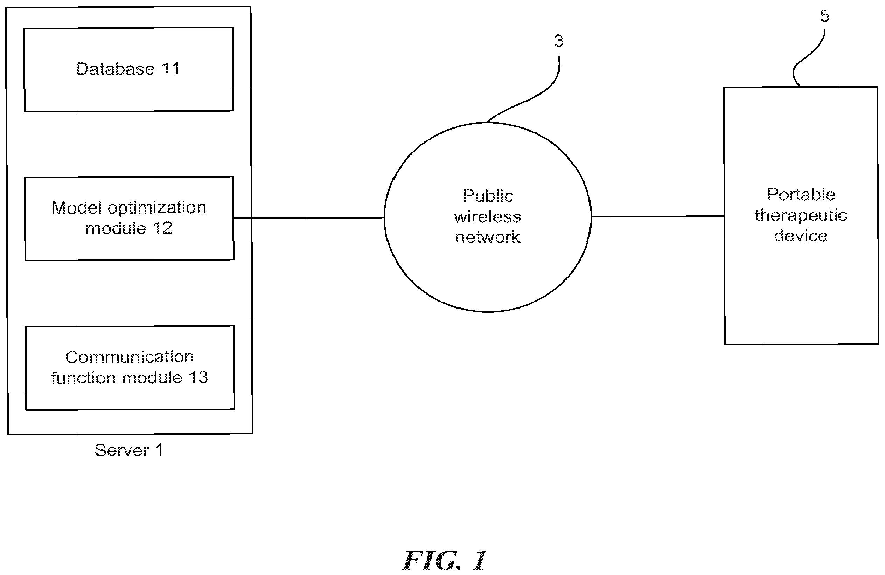

Referring to FIG. 1, the present invention provides a therapeutic system using customized bioelectromagnetic waves varying dynamically with time for excitation, which includes a portable cell or tissue excitation terminal or device 5 and advantageously a separate external computer, computer system, or computer server 1. The terminal or device 5 may be referred to herein as simply the portable device 5 or as the cell or tissue excitation terminal. Advantageously, the system is configured for communication with a remote or external computer or server that provides system and device operating information and signals, waveform characteristics, patient treatment data and history, statistical and other information and analytics processing capabilities for analyzing individual patients, multiple different patients, illnesses and condition response to different treatments, as well as other information and/or processing. The external computer or computer server 5 is advantageously used in examples of the system to provide for additional processing, execution of computer programs for waveform generation and analysis, analysis of patient user data and information, optimization, and other analytic and storage functions that if included in the terminal device 5 would result in that device being unnecessarily expensive. Furthermore, it is technically advantageous to physically separate the EM wave emitter from computational circuit elements, executing processors, data storage, and the like so that EM radiation interference is reduced.

In one non-limiting example, the portable cell or tissue excitation terminal or device 5 may operate autonomously and have memory storage and processing logic that is sufficient to provide the diagnostic and therapeutic operations described herein. Alternatively, the portable cell or tissue excitation terminal or device 5 may be intermittently connected or coupled with an external database and/or computer or server to provide the described diagnostic and therapeutic features. A configuration of the portable cell or tissue excitation terminal or device 5 may be chosen with manufacturing and sales price in mind and where a lower cost device 5 is advantageously, the system configuration with a server may be advantageously adopted. It will be appreciated that even when a server is configured, a cloud-based server may be employed to provide access to additional processing and/or data storage and retrieval.

In another non-limiting example of the portable excitation terminal or device, the device provides a case or housing that contains and includes a wireless front-end for wireless communications with antenna, a micro-controller or processor logic, an accelerometer or other heartbeat or pulse sensor and optionally other sensor, a coder-decoder (CODEC) circuit or computer program encoding and/or decoding a digital data stream or signal, a waveform shaping and generating unit or engine, a power driver unit coupled with the control integrated circuit (IC) or processor or microprocessor, or CPU 50, and a magnetics (or electromagnetics) unit that includes an inductor element that is responsive to a current signal from the Power driver to emit the EM field.

In one non-limiting example, when a server or server function is provided, the server 1 is connected or coupled to the cell excitation terminal 5 through a communications link such as through a wired and/or wireless private or public wireless network 3. A wireless coupling from the cell excitation terminal 5 is preferable because if permits unencumbered movement (no wires or cables) between the cell excitation terminal 5 and any other system or network component; however, it will be appreciated in light of the description provided here that a wired connection or coupling may be used for communications with the cell excitation terminal 5. The network, such as a public wireless network 3 may be a common wireless communication network or Internet. Other wireless technologies, such as WiFi, WiMAX, Bluetooth, or other radio-frequency or optical wireless communication links or means may be used. Furthermore, various different wired and/or wireless communication links may be used in combination for communications between the cell excitation terminal 5 and any external entities, such as the server 1.

The server 1 may be a private or proprietary server platform or a public service platform, and can provide services for multiple patients or users at the same time. The server 1 includes a database 11 (or may be coupled to or with separate databases), and an optimization module 12. The portable unit or cell excitation terminal 5 is a small sized micro therapeutic apparatus or device 5, can be conveniently located or fixed on a part of a human body and perform therapy (EM radiation) on the part, and can be used as a part of the remote therapeutic system or separately. In one non-limiting example, the small sized micro therapeutic apparatus or device 5 is sized and shaped to fit within and conform to the human hand so that EM wave emission out the surface of the device 5 may be directly radiated into the biological tissues of the palm of the hand. In this particular example, the device 5 has a size of less than about 10.16 cm by 5.08 cm (4 inches by 2 inches), while in other examples, the device 5 may be smaller, for example on the order of approximately 2.54 - 7.62 cm (1-3 inches) in any dimension. The emission area is advantageously between about 1.27 cm (½ inch) and 5.08 cm (2 inches), though larger or smaller emission areas by be utilized. Therapeutic treatment may optionally include manually moving or manipulating the location of the cell excitation terminal 5 over a patient body part or region. The server is connected to the public wireless network 3 through a communication function module 13.

The inventive cell or tissue excitation terminal or device may be made to suit the particular treatment application.

In one non-limiting example, the portable excitation terminal device or unit has several specific functions that may include: (a) means for receiving the encoded (encoding is optional) waveform data, for decoding it (only when optionally encoded), and for converting the data into an analog electro-magnetic field (including field strength, waveform shape, waveform period). It also includes (b) means to radiate and focus the electromagnetic field into the patient's body, and (c) means to measure or sense the patient's heartbeat and use it as a feedback to fine tune the magnetic field and in some treatment regimes to modify or control or attempt to modify or control the heartbeat. Encoding may be used either to maintain a privacy or security or proprietary interest in a particular signal waveform, or to provide data compression, or for other reasons or combinations of reasons.

The portable unit, also or alternatively referred to as the cell or tissue excitation terminal or device, includes a control integrated circuit (control IC) such as a processor, processing logic, or Central Processing Unit (CPU) 50. The control integrated circuit, processor, processing logic circuit or CPU may advantageously intemperate with firmware and/or software that modifies and controls the operation of the portable unit under programmatic control. This control integrated circuit component provides:

(a) a Radio Frequency (RF) communications interface

(b) a data processing element, and where encrypted information is to be communicated, such as customized waveforms that may be proprietary and/or patient health data that may be protected, the processing element may include encryption and decryption processing features or element,

(c) a digital-to-Analog (D/A) conversion module or unit for converting digital waveform information into an analog signal for driving the inductive emitter element,

(d) a power generation circuitry, and

(e) a sensor or detector for sensing patient physiological status, such as an accelerometer, Infrared detector, or other heartbeat or patient pulse rate sensing device or other sensing means;

The control integrated circuit (control IC) such as a microprocessor, processor, processing logic, CPU or other processing means is the device's brain or intelligence and will manage the overall processing, system timing, RF, communications, and the sensor(s) such as the heartbeat detector, pulse detector, skin moisture detector, blood oxygenation detector, temperature detector, or other sensor. Additional intelligence may be provided by a coupled or intermittently coupled external processor such as another computer or server.

The waveform shaping section will smooth the output signal from the control IC processor, and may in some examples also be responsible for level shifting the voltage level to match the power driver stage.

Combining the power driver stage with the magnetics unit or inductor element(s) generate the requisite electromagnetic field. Since the field strength is related to the current level flowing through the magnetics or inductor element(s) so the electromagnetic field strength of the portable excitation terminal is controlled at least in part by providing an appropriate current signal, such as I(t) as described in greater detail elsewhere herein, in the current path. In one example, the inductor element is designed so the electromagnetic field is radiated perpendicular to the plane of a printed circuit board (PCB) that carries or mounts the several circuits and devices within the portable exciter terminal or device 5 so the EM wave field will be radiated from the flat face of the portable handheld unit and into the body portion.

In one non-limiting example, the electromagnetic field strength radiated by the unit will range from approximately 10 micro Tesla (10uT) up to 325 micro Tesla (325uT), though these field strength examples are not limitations of the invention and higher or lower levels may be applied. Patient body characteristics, identified sickness, disease, or condition as well as sensor feedback may suggest higher or lower filed strengths for appropriate beneficial treatment efficacy.

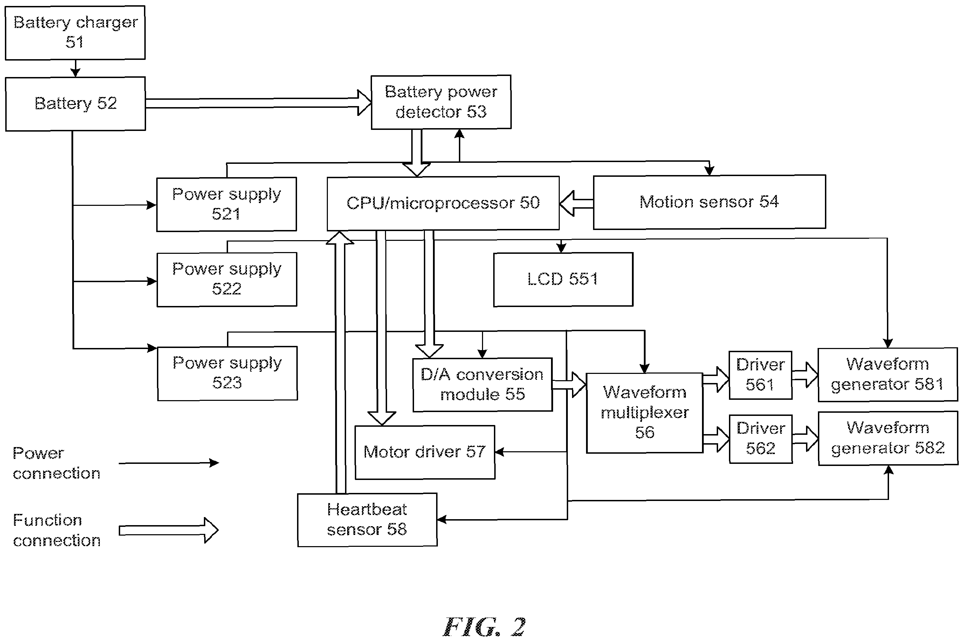

Referring to FIG. 2, the portable device or cell or tissue excitation terminal 5 according to an example the present invention includes a processor, microprocessor, processing logic circuits, or central processing unit (CPU) 50, a human body status detection device or sensor connected to the CPU 50 and is used to monitor human body or biological tissue status, a device for communication with the outside, one or more bioelectromagnetic wave generators, and a power device for providing the devices with power. References to a CPU in this patent is intended to mean or include a processor, a microprocessor, a processing logic circuit, a central processing unit (CPU), or combinations of these. The processing logic may be hardware and may include firmware and/or software (executable computer program instructions as well as data) to control and/or change the operation of the hardware. The (when only one) or each (when more than one) of the bioelectromagnetic wave generators 581, 582 is connected to the CPU 50, and the CPU 50 controls, according to a signal detected by the human body status detection device (such as the Heartbeat sensor or detector 58), the bioelectromagnetic wave generator to send bioelectromagnetic waves corresponding to a detected or sensed status or subject patient. Although two wave generators 581, 582 are illustrated and described for this particular embodiment, it will be understood that other embodiments have only a single wave generator and also may not require such complex waveform selection or multiplexer circuits or logic. One embodiment provide only a single waveform generator that has the ability to generate many different waveform characteristics.

In this embodiment, the device for communication with the outside is not shown in FIG. 2, and is a common communication device, for example, an antenna and a relevant communication module of a mobile phone connected to the wireless communication network, or a wired or wireless network connecting device connected to Internet. Bluetooth, WiFi, WiMAX, or other wireless or wired communication means may alternatively or additionally be used.

The power device adopts a battery, for example a common lithium-ion battery, and includes a battery charger 51, a battery 52, and power supply modules 521, 522, and 523. An external AC charger will provide power to the internal battery charger 51 to charge the battery 52 to guarantee continuous supply of power of the battery 52. Replaceable batteries may alternatively be used. The battery 52 supplies power to components of the cell excitation terminal through the power supply modules 521, 522, and 523 respectively. It will be understood in light of the description provided here that a single power supply may be used so long as that single supply provides an appropriate voltage and current to provide operating power to satisfy the power requirements of the device 5 circuitry. The battery charger 51 is connected to external power through a power connection port 531, so as to perform charging.

For embodiments of the portable excitation terminal that provide two waveform generators, the EM wave generator is formed by a waveform multiplexer 56, drivers 561 and 562, and waveform generators 581 and 582 connected to the drivers 561 and 562. The waveform multiplexer 56 is also common equipment in the market, receives the digital signal representing waveforms output by the D/A conversion module 55, which processes and converts the digital signals from the CPU into the required analog signal. In this embodiment, two sets of EM wave output equipment are included. The waveform multiplexer 56 can output two waveform analog voltage signals that are input to the drivers 561 and 562 respectively. The drivers 561 and 562 respectively drive the waveform generators 581 and 582 to generate the corresponding bioelectromagnetic waves. The waveform generators 581 and 582 may adopt coil inductors to generate EM waves of a certain voltage. In this embodiment, EM waves in two paths are generated, and the two kinds of EM waves may have the same waveform or different waveforms. In addition, also according to needs, it may be designed to generate EM waves in one path or more than two paths, and one or more drivers may be designed. The waveform generators 581 and 582 are configured to be able to use a relatively low-frequency pulsed magnetic field to stimulate blood circulation in the body of a patient. The low frequency normally ranges from 10 Hz to 200 Hz, typically ranging from 20 Hz to 120 Hz, more typically ranges from 20 Hz to 40 Hz, and is about or actually 30 Hz (for example, $30\pm3$ Hz) in a specific case.

Other examples of the portable excitation terminal have only a single wave generator and do not require the waveform multiplexer 56 or the second driver 562.

The central control unit 50, which may be a integrated circuit such as a microprocessor or a single-chip microcomputer, is the control center of the cell excitation terminal and controls the operation of the rest of the components in the device. The D/A conversion module 55 performs the conversion of digital representation of the waveforms generated by the central control unit 50 into analog waveform, which is then used by the driver circuitry to excite the waveform generators, thus generating the desired EM field.

In this embodiment, the human body status detection device is implemented as a heartbeat sensor 58. The heartbeat sensor 58 can measure a heart rate of a human body, and input the heart rate to the central control unit 50. The central control unit 51 may determine current physical status of a patient according to parameters read by the heartbeat sensor to determine a required therapeutic EM waveform or waveform combination and a therapeutic manner, so as to send control signals (digital signals representing waveforms) to the D/A converter, waveform multiplexer 56 and the drivers 561 and 562, so that the waveform generators 581 and 582 respectively generate a determined EM wave or EM wave combination to perform radiation therapy on the patient according to the determined manner. Similarly, the human body status detection device for measuring the physical status of the human body may also be a motion and acceleration detector, blood pressure detector or sensor, a human body blood viscosity detection device, an electroencephalogram (EEG) measurer, an electrocardiogram (ECG) measurer, or other apparatuses or devices capable of detecting the real-time physical status of the human body conveniently. One or more of the apparatus or devices may be used to provide a set of data of the human body status.

The motion sensor 54 may use or adopt a motion accelerometer for measuring the heartbeat and/or pulse. Alternative implementations use an Infrared (IR) based detector to sense or misuse the heartbeat and/or pulse. When used, the accelerometer is a highly sensitive motion sensor. When a patient or a user holds the device by hand and stays still, the sensor detect most small vibrations (such as heartbeats and blood flow pulses) in the human body, a heart rate spectrum of the patient is embedded in a vibration signal, detected signals are input to the central control unit 50, and the heat rate spectrum or pulses of the user are detected and extracted from all of the signals through digital signal processing (DSP) technologies.

When the sensor for sensing or detecting a heartbeat rate or timing (an optionally the intensity or strength of the heartbeat or pulse) is used is an accelerometer it may be appreciated that such accelerometer is a very sensitive motion sensor and when the patient holds the device in their hand while resting, it will be able to detect most of the minute vibration (such as a heartbeat or blood flow pulse) from the patient's body, and the patient's heartbeat pattern will be embedded into the vibration signals. Other or different sensors that provide status information or feedback for adaptation of the emitted pulses may be used, such as the IR sensor described elsewhere herein.

In one embodiment, the portable device may be attached to the body at the point where the EM wave is to be applied in such manner that the accelerometer can sense the pulse of blood flowing nearby and use this sensed signal. Tape or straps may be used for the attachment, or in some instances the weight of a towel or other covering may be sufficient to maintain contact to facilitate accelerometer sensed vibrations or pulse. The portable excitation terminal or device control integrated circuit, such as the processor or CPU 50, may be responsible to interpret the signals from the accelerometer and through Digital Signal Processing (DSP) techniques it will detect and extract the patient's heartbeat pattern or pulse from the overall accelerometer detected or generated signal. For example, although the accelerometer may detect other patient movement, the vibrations detected from such movement are of different characteristics or frequency and may be filtered out using the signal processing techniques.

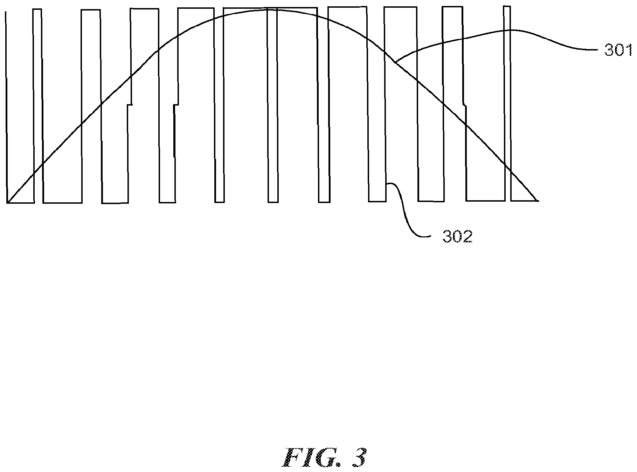

Many methods may be used to generate different bioelectromagnetic waveforms. FIG. 3 illustrates a Pulse Wave Modulated (PWM) waveform with a duty factor (black) that is varying with time, and illustrates a sinusoidal or sine wave (a smooth curve) formed after low-pass filtering.

FIG. 3 shows a pulse wave modulated PWM waveform with a time varying duty cycle (pulses) juxtaposed against the sine wave formed after low-pass filtering or a Digital-to-Analog conversion processing (smooth line). It should be appreciated that a variety of different waveforms may be generated from and/or synthesized the PWM signal in combination with the smoothing where by low-pass or even band-pass filtering or by other Digital-to-Analog conversion, and the use of Digital Signal Processing (DSP) and/or analog processing techniques to form different waveforms from PWM waveforms of this type are known generally even though the formation of particular therapeutic waveforms for use in generating and emitting EM field for treatment was not heretofore known. If the duty cycle of the PWM waveform stays constant, a DC level will be formed after low pass filtering. If the duty cycle is not constant then a varying DC level may be created. The duty cycle may be adjusted in accordance with the waveform so that the DC level or bias is zero.

A simple and effective low-pass filter can be formed by using an resistor-capacitor pair. Combining PWM and a low-pass filter forms a cost effective digital to analog converter, providing an accurate and simple wave shaping and synthesis function. Waveforms of different shapes, frequencies, and amplitude may be generated by combining a PWM system with a low-pass or even high-pass filter. More advanced D/A converters can also be used in the terminal to perform even higher accuracy waveform synthesis. It may also be appreciated that a D/A system may be used to form the waveform from other input signals and the use of the PWM with filtering is not a limitation of the innovation. The PWM and low-pass filtering or D/A conversion is only example of how the input(s) to the driver may be implemented to realize a D/A function.

The central control unit 50 can send human body status information to the server 1 through the communications module. The central control unit 50 can customize a bioelectromagnetic waveform suitable for a specific patient based on the downloaded information of the user's human body status information and other physiological and medical parameters that are stored in the database 11. These parameters can include therapeutic history, known sickness, electrical properties of the patient's body, and other specific factors, such as skin thickness, amount of fat and tissue under therapeutic locations, heart rate, and/or other physical factors.

A liquid crystal display (LCD) 551 is used as the user interface to display the working state of the whole cell excitation terminal or working parameters during the excitation phase of the terminal.

An optional function connection port 532 is used for external connection, such as for example a universal serial bus (USB) port, so as to provide and input/output (I/O) interface for input data or download a program from the outside. This may be provided instead of or in addition to any wireless communications available with the device.

The power connection port 531 is used to connect the power to supply the power to the portable device, such as for charging an internal battery.

A battery power detector 53 is used to monitor a power use situation of the whole system. In addition to providing an ability to monitor and detect a low battery situation that may suggest that the battery power is getting low, the battery power detector 53 is advantageously provided as a safety feature so that a user or therapist is not able to operate the device 5 when the device 5 is not able to generate the intended beneficial therapeutic waves for treatment. In other words, the battery power detector 53 is configured to disable the device so that treatment cannot start and no uncontrolled EMF emissions can occur that would or might harm the patient, or that would not result in the intended treatment. As described elsewhere herein, the device 5 may include a predictive feature so that the processing of the CPU 50, and indication or stored history of the battery usage, and/or a smart battery circuit that is able to identify how much charge is remaining, are taken into account as well as the intended treatment, so that a signal or indication may be provided that the remaining battery power is not sufficient for the intended treatment.

Referring to FIG. 1 and FIG. 2, the cell excitation terminal 5 and the server 1 communicate through the private or public wireless network (such as the public Internet or any private intranet), thereby forming a complete therapeutic ecosystem. The server 1 may advantageously include all, some or a combination of the following functional modules.

(1) The database 11. The database 11 is used to store information and parameters relevant to each patient. The general waveforms repository, a mapping of the relationship between patient sickness or treatment condition relative to the applicable waveforms, patient's physiological history, and other physical or status parameters can be included.

(2) The waveform or treatment model optimization module 12. The waveform or treatment model optimization module 12 constantly reviews and analyzes the treatment results and efficacy of any or all patients to intelligently select the best treatment scheme for any patient. A treatment scheme can include waveform shape and strength selection, treatment time period, and number of treatments. This constant optimization process ensures that the treatment efficacy can be optimized and the therapy can achieve the desired beneficial effects. Implementation of the optimization module and algorithms according to the aforementioned process may also be performed by persons skilled in the art of designing relevant software or hardware.

(3) The communication function module 13. The communication function module 13 communicates with the cell excitation terminal 5 through a communications link or interface, such as the public wireless network 3. It may be appreciated that many different types of communication devices, protocols, signaling schemes, are known in the art and may be applied to this communication. A cell phone (mobile phone) may also be usefully used as the module or a bridge for communications or connections. The cell phone can forward the information received by the server 1 to the cell excitation terminal 5 wirelessly through a local radio frequency (RF) channel that is based on Bluetooth, or other wired or wireless connections.

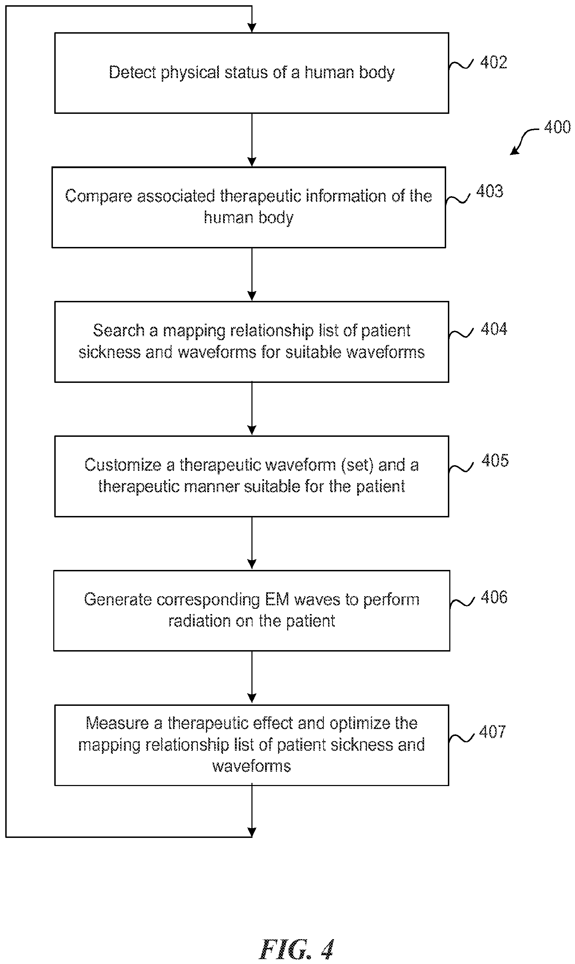

Referring to FIG. 4, the central processing unit or control unit 50 acts as a central control device for the whole therapeutic system, controlling the operation of all the components in the system to perform treatment on a patient, and to ensure a therapeutically effective and safe dose of electromagnetic radiation. The workflow is as shown below.

In Step 401, the process is initiated including for example, loading or verifying the waveform to be applied to the body part are loaded and ready, placing the excitation terminal device at the treatment location, and initiating or starting the treatment with the initial waveforms.

In Step 402, a sensor detects a physiological status of the human body or other animal or tissue undergoing treatment. In one non-limiting example, the heartbeat sensor 58 is instructed to detect human body status (such as pulse rate), and send the detected human body status information to the database 11 of the server 1 through the communication module.

In some non-limiting examples, information may be stored in a memory of the device, such as in a separate solid-state memory or in a memory associated with the CPU, so that for some situations, communication with the server 1 and/or database 11 may not be required and the treatment decision is made locally by the CPU. It may further be appreciated that other processing that is described as performed at the server 1 and/or with the database 11 may be performed entirely by or in part by the device 5; however, the greater processing power and storage could result in a more expensive portable device. In some non-limiting example, the external server and database may be a small computer, such as a Personal Computer, or a hand-held computer such as an Apple iPhone, iPad, or other smart phone that includes sufficient processing power, database storages, application software, and the like to interoperate with the portable device 5.

In Step 403, a comparison is performed between the associated therapeutic information on the patient that was stored or measured and other associated therapeutic information stored locally or on server 1, so as to determine a sickness or treatment condition as well as a beneficial therapeutic treatment plan or stage.

In Step 404, a therapeutic waveform or waveform combination and a therapeutic means and/or regime are searched for and identified using parameter comparison, pattern matching, or other statistical or analytical techniques using the mapping relationship list or data structure of patient sickness or treatment conditions and waveforms in the database 11.

In Step 405, a waveform or waveform set are selected from available waveforms or constructed as a customized waveform or waveform set, such as by way of example but not of limitation, by instructing the D/A converter (and any waveform multiplexer 56 that may be present in embodiments having multiple waveform generators) to output a particular waveform signal(s), which are respectively input to the driver 561 and/or driver 562. When only a single waveform is selected and/or generated, the waveform multiplexer 56 is not required and only one driver 561 is provided.

In step 406, the selected waveforms are input to the driver 561 (and/or driver 562 when provided) so that the waveform generator 581 (and driver 582 if present) generates a corresponding EM wave (or EM wave combination) to perform the therapy on the patient in a therapeutic manner, for example, according to a set time period or cycle. It may be appreciated that only a single waveform may be identified and/or provided or that two or more (a plurality) waveforms are identified and provided and that the example shown in FIG. 2 represents only an example configuration. For embodiments, having only a single waveform generator, the multiplexer and selection are not used, and otherwise the method or process described here is the same but using only the one waveform generator and driver.

In Step 407, a therapeutic effect from the current and preferably from prior treatments (if any) is measured and assessed, and data is input to the optimization module 12 to optimize the mapping relationship list or other data structure of patient sickness or treatment condition and corresponding waveforms. It may be appreciated that the optimization module may be performed either in a separate circuit, processor, and/or computer program software implementation or alternatively or in addition be performed in the processor, microprocessor, processing logic, or CPU 50 present in the portable excitation terminal device 5.

In Step 408, optionally but advantageously, a preferred therapeutic scheme is selected by performing processing, selection, and/or filtering of candidate waveforms, according to the therapeutic effect of the patient within a certain period of time, and the mapping relationship list of patient sickness or treatment conditions and/or other factors and waveforms is optimized. This step may occur after the first treatment so as to optimize the next treatment either in the same treatment session or in a subsequent treatment session.

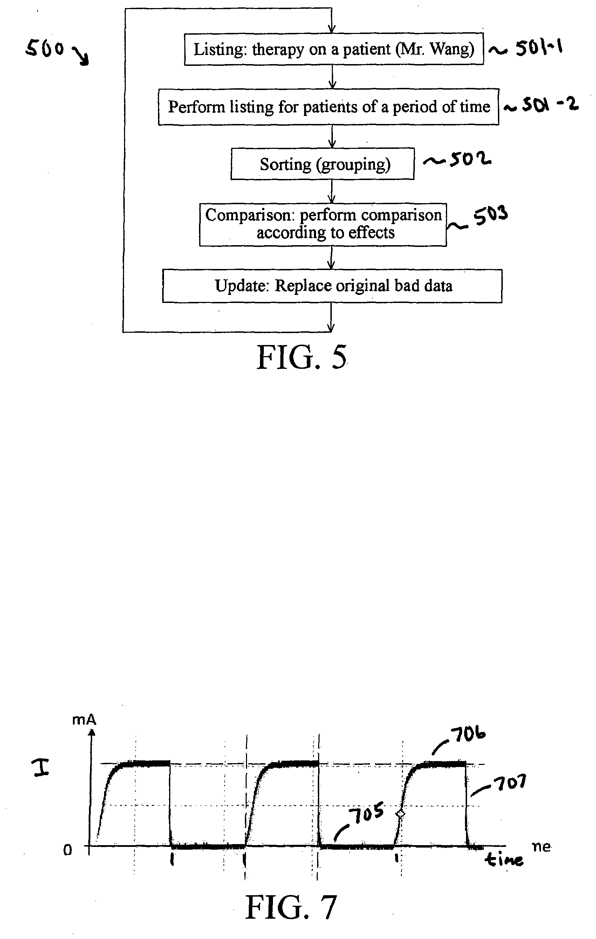

FIG. 5 illustrates an embodiment of an example optimization process 500 of the "mapping relationship list of patient sickness or treatment condition relative to the applicable waveforms". Referring to FIG. 5, an update process is as follows.

Listing (Step 501) is performed as follows. A list is prepared, which advantageously includes sufficient information to associate and select at least one waveform that is a best match for treating a patient sickness or other condition. In one non-limiting example the list or table includes "sickness or treatment condition", "therapeutic waveforms", "a therapeutic means" of a therapeutic scheme of a patient (for example Mr. Wang) within a period of time (for example, 30 days or other time period), and therapeutic effects which are classified into grades. For example the therapeutic regimes may be numbers on some scale (such as a scale of 1-10) or using labels, such as for example "excellent", "obviously effective", "so-so" or "some-what effective", and "ineffective", according to recorded human body status information and according to a set or predetermined standard. Meanwhile, the parameters of various patients (such as Mr. Zhang, and Mr. Li) of a certain period are also listed in the list or table.

Sorting or grouping and selection (Step 502) is performed as follows. For a certain period of time, for example 30 days, data of all patients of the same sickness or condition are grouped in the list, data parameters corresponding to "excellent" and "obviously effective" or other ranking scheme in each group are selected as these are now known to be useful for achieving a beneficial therapeutic effect.

Comparison (Step 503) is performed as follows. The data parameters corresponding to "excellent" and "obviously effective" or other ranking scheme and selected during the sorting are compared with a therapeutic effect in the current list.

Update (Step 505) is performed as follows. The "therapeutic waveforms" and "therapeutic means" of the sickness or condition corresponding to the compared data parameters being good or effective are used to replace the original "therapeutic waveforms" that may have been less effective or not effective and "therapeutic means".

In one non-limiting example, the above process 500 may be performed constantly or repetitively and predetermined or dynamically determined time intervals, such as in a circulating and repeating manner, so that an optimized therapeutic scheme and dosage are applied. In some non-limiting examples, the updating will occur during the emission and delivery of EMW to the patient so that the waveforms are constantly updated. In other non-limiting examples, they may be updated between treatments, or the treatment may be paused or suspended and emissions stopped while an update is made, and then the therapy resumes with the different waveforms..

Functions of the processor, processing logic, microprocessor, central processing unit, or central control unit 50 are already described above clearly. However, for persons of ordinary skill in the art, the central control unit 50 as a functional component may reside in the cell excitation terminal 5, or separately reside in the server 1, or integrated together with other components, for example, the database 11. In some non-limiting examples, a portion of the central control unit 50 may reside in the device 5 and more processing intensive operations relying on a processor or supplementary processor in an external unit such as in a separate computer or server.

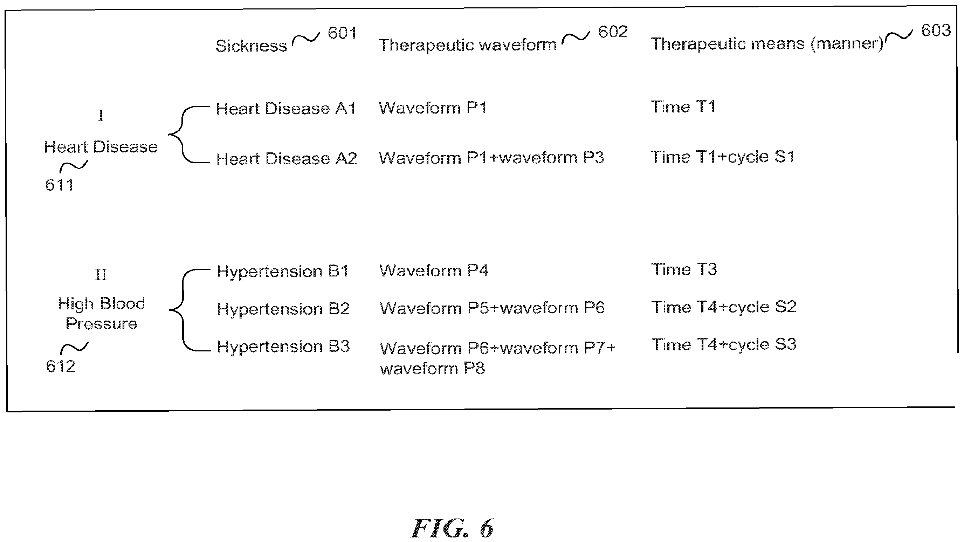

FIG. 6 is a schematic view of an embodiment of the mapping relationship list or table of patient sickness or condition and waveforms. The list is formed by "sickness", "therapeutic waveforms", and "therapeutic means". The sickness is in a left column, and involves various sickness capable of being treated, for example, a heart disease, a high blood pressure, high cholesterol, and a headache.

The heart disease may be divided into heart diseases A1 and A2 according to ages and the sickness. The high blood pressure may also be divided into high blood pressures B1, B2, and B3 according to ages and sickness or according to other factors.

A middle column indicates therapeutic waveforms including P1, P2, and P8, which may be a single waveform, or a waveform combination of two or three (or more) waveforms, and waveform features, such as intensity, of each of the waveforms may not be the same. A right column indicates a therapeutic manner or means, for example therapeutic manners such as a continuous therapeutic period of time including T1, T2, T3, and T4, an intermittent therapeutic cycle including S1, S2, and S3, and a frequency indicating cross-use of various waveform combinations.

According to the list shown in FIG. 6, the central control unit 50 (such as CPU within the device 5 or in combination with an external processor) can customize a preferred therapeutic manner corresponding to certain sickness or condition according to information such as physical status of a patient.

Therefore, it can be seen that the therapeutic manner can optimize and in many instances guarantee that a preferred therapeutic effect can be achieved by combining the time, cycle, and frequency and by radiation of various therapeutic waveforms and therapeutic waveform combinations.

As described elsewhere herein, the therapeutic manner also results from the optimized mapping relationship list of patient sickness or condition and waveforms. Advantageously, the optimization is constantly updated or at least updated at frequent intervals. The optimization is based on assessment, feedback, and analysis such as using comparison, selection and filtering performed on several therapeutic schemes, thereby constantly optimizing the therapeutic effect and ensuring the therapeutic effect to be reliable and accurate. It may be appreciated in light of the description here, that this updating and optimization is a major difference between the present invention and the conventional prior art, makes a breakthrough in the EM radiation therapeutic manner and a therapeutic effect of the EM radiation therapeutic manner, and is achieved through creative efforts of the inventors of the present invention.

The therapeutic bioelectromagnetic waveform according to the present invention depends on the sickness or condition of the patient and various items of human body status information, such as heartbeat rate, pulse rate, blood pressure, stress level (possibly as measured by a skin moisture detector or sensor), blood oxygenation, level and/or other biological or body vital signs or conditions alone or in any combination. The waveforms have different shapes, cycles, and intensity, which are all listed in the mapping relationship list or other table or data structure of patient sickness or condition and waveforms and are updated from time-to-time.

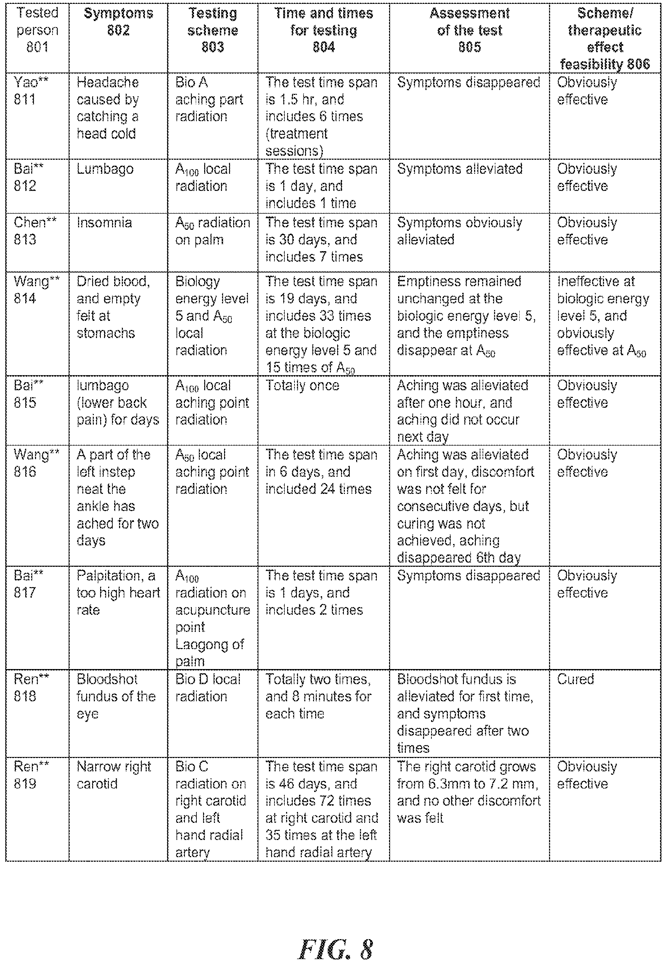

FIG. 8 provides a table identifying various tested person, the symptoms exhibited or described by the tested person ###, a testing scheme, a time and times for testing, and assessment of the test, and a change or therapeutic effect. The table of FIG. 8 identifies various sicknesses various sickness, , such as a headache, stomach ache, cold, cough, insomnia, and lumbago.

The example waveform shown in FIG. 7 is a result from therapy performed in a certain period of time in respect of various sickness shown in FIG. 8. According to the therapeutic result, an embodiment of the waveform shown in FIG. 7 has a therapeutic effect on the identified sickness or symptoms of the sickness or condition, such as for example on headache, lumbago, and cold or influenza, thereby being "obviously effective" (achieving an obvious therapeutic effect).

In addition to the example waveform of FIG. 7, it will be appreciated that a great variety of different waveforms may be constructed using the simple D/A converter consisting of a PWM digital pulse stream, and resistor-capacitor circuit or RC filtering, and then driving the waveform generator as described to generate the current waveform I(t). In one non-limiting example, the waveform generator that participates in generating the emitted electromagnetic wave (see for example the waveform generators 581, 582 of FIG. 2) may include at least a physical inductor usually in the form of a wire coil that has at least some electrical resistance as well as the inductance. Tuning for particular inductor characteristics may be achieved by selecting the physical inductor properties to achieve the desired characteristics including the time constant. Wire passing a current may be coiled in order to concentrate the magnetic field generated with the size of the wire, the number of coils, the diameter of the coils (inner and outer diameter if coiled in the form of an annulus) and the thickness of the coil being possible factors in defining the inductance and its inductive element properties. It may be appreciated that different excitation terminal devices may use different coil properties in combination with the waveforms in order to achieve different effects. Furthermore, multiple or a plurality of same or different inductive elements may be used in a single excitation terminal and be energized at the same or different times (perhaps switching between them during a single treatment session or available at different times for different treatments) in order to achieve the desired EM wave emission(s) and optimal therapeutic benefit.

As is known in the electrical art, inductance is the ability of an inductor to store energy in a magnetic field. An inductor generate an opposing voltage proportional to the rate of change in current in a circuit. The standard definition of the self-inductance L of an electrical circuit (in webers per amp, referred to as henries, but any consistent set of units may be used) is voltage equals the self-inductance L multiplied by the change in current divided by the change in time or $v = L \, di/dt$, where $v$ denotes the voltage in volts and $i$ the current in amperes.

It will be appreciated therefore that the system and device according to examples of the innovation described here are adapted to generate and utilize a great variety of different waveforms, and that it would be impractical to describe all of the possible waveforms here.

However, one general form of a particularly useful waveform is a waveform that has been found to be a therapeutically effective waveform generated by the waveform generator of the form:

$$I(t) = \begin{cases} 0 & , \ t < 0 \\ I_{max} \times (1 - (1/e^{t/\tau})) & , \ 0 \leq t \leq t_1 \\ 0 & , \ t > t_1 \end{cases} \qquad \text{Equation (1)}$$

where

a) 5 msec $\leq t_1 \leq$ 0.1 msec
b) 0.3 msec $\leq \tau \leq$ 20 msec
and

- $\tau$ is a time constant of generation of the waveform generator,

- $t$ is any time point,

- $(t_1 - t)$ is a duration or on-time of a pulse, and

- $I_{max}$ is a maximum direct current (DC) following in the circuit.

In accordance with one example of the system, device, and therapeutic application including both diagnostic and treatment features that can be used separately or in combination, it has been determined that the system, device, and method, a portable device that generates PEMF, can provide significant benefits to a patient's cardiovascular system.

More specifically, the device, system, and method have been found to improve the condition of patients with Hypertension (high blood pressure) which is a cardiac chronic medical condition in which the systemic arterial blood pressure is elevated or too high. as well as Hypotension (low blood pressure) in which the systemic arterial blood pressure is too low.

With hypertension, the heart has to work harder than it should to pump the blood around the person's body. Blood pressure involves two measurements, systolic pressure and diastolic pressure. Normal blood pressure is usually regarded as a systolic/diastolic pressure of 120/80 mm/Hg. The systolic blood pressure is the pressure in the arteries when the heart muscle is contracting. The diastolic blood pressure is the pressure in the arteries between heart beats when the cardiac muscle is relaxed or not contracted. It is generally recognized that high blood pressure is anything above systolic/diastolic pressure 140/90 mm/Hg. Hypotension is the opposite of hypertension.

Persistent hypertension is one of the risk factors for stroke, myocardial infarction, cardiac or heart failure, and arterial aneurysm, and is a leading cause of chronic kidney failure. Other and related coronary heart disease (including for example, but not limited to coronary artery heart disease involving myocardial ischemia, and arrhythmia and ventricular fibrillation) may also develop, and there may be other ill effects from hypertension and hypotension.

In terms of blood pressure improvements, experimental data have indicated that the device, system, and method are able to beneficially alter or correct blood pressure (high or low) within substantially a ten percent (10%) range or window, and more usually within a 5% to 10% range or window, with particular results indicating a 8% range or window. More of different beneficial improvements may be realized with other examples of the device and method and with further refinement and/or customization in the signals. For example, continued research and development are expected to modify blood pressure to even a greater range, such as by as much as 20%, and possibly more with additional research and development and/or in conjunction with other treatment regimes. Even an 10% reduction in a hypertensive person having an untreated systolic/diastolic pressure of 140/90 mm/Hg and having a beneficial reduction of 10% would achieve a systolic/diastolic pressure of 126/81 mm/Hg moving that person out of the recognized hypertensive range or prehypertensive range, a further reduction would provide an even further benefit. The improvement is blood pressure is in addition to other realized benefits.

By way of example, but not of limitation, the radiated PEMF may be generated by a repetitive current waveform, and a single cycle of the current waveform as a function of time I(t) can be defined by the following Equation (2):

$$I(t) = \begin{cases} 0 \ , \ t < 0 \\ I_{max} \times (1 - (1 / e^{t/\tau})) \ , \ 0 \le t \le t_1 \ . \\ 0 \ , \ t > t_1 \end{cases} \qquad \text{Equation (2)}$$

Where:

e is the exponent function;
$\tau$ is equal to the time constant created by an inductor element in the driver circuit;
t is any time point or point in time;
$(t_1 - t)$ is the duration or the ON-time of the pulse, and
$I_{max}$ is the maximum DC current that can flow through the driver circuit.

In accordance with one non-limiting example, the maximum current ($I_{max}$) is the maximum sustainable current that the circuit can provide to the driving circuitry. More importantly, it represents the maximum current achievable within the time constant ($\tau$), which is relevant to the output power of the circuit as indicated by Equations (2) and (3).

In order to achieve the maximized or optimized or at least near-maximized or near-optimized therapeutic efficacy or at least a therapeutically effective amount for beneficial improvement of the patient's cardiovascular conditions, the following factors are advantageously considered and adjusted or optimized to the extent possible:

(a) the point of application of the Pulsed EMF (PEMF);

(b) the frequency of EMF pulse switching for example, frequency in oscillations or cycles per second or Hertz (Hz); and

(c) the magnetic field strength of the EMF at the point of application (or at some other reference point)

Due, for example, to varying physiologies of patients, a range of frequency of the EMF pulse switching and of the EMF magnetic field strength are advantageously considered to achieve the desired therapeutic benefit for each patient and for different points of applications to the body or body tissue. It has been determined that application of PEMF within the defined range of frequency and field strength maximizes the therapeutic effect, while application of frequencies and/or field strengths beyond the defined ranges will have minimum beneficial therapeutic effect or no beneficial therapeutic effect to the patient's cardiovascular system.

It may be appreciated that the reference above is to the magnetic field strength (measured in tesla or microtesla) as compared to an electric field strength that is or may also be generated. The difference is that a force of magnetic field on a charged particle is generally due to the charged particle's movement while the force imparted by an electric field on a charged particle is not due to the charged particle's movement.

Non-limiting examples of the system, device, and method may provide therapeutic treatment benefits based on the magnetic field applied, the electric field applied, and/or on the combination of magnetic and electric field applied.

The ranges of PEMF frequency and field strength that provide the beneficial therapeutic effect are as follows:

(a) EMF pulse switching Frequency range: generally between about 10 Hz and about 75 Hz, and more particularly between substantially 15 Hz to substantially 60 Hz, where the optimum switching frequency may usually be dependent on characteristics of the patient tissue and/or body part or area where the pulsed EMF is applied.

(b) Magnetic Field Strength range: generally between about 25 uT (micro Tesla) and 500 uT, and more particularly between substantially 50uT to 300uT, and even more particularly in the range between substantially 100 uT and 200uT. The tesla (T) is the international system unit (SI) derived unit of magnetic field B (which is also known as "magnetic flux density"). One tesla is equal to one Weber per square meter.

It may be appreciated in light of the description provided herein that the actual setting of the PEMF device and/or system characteristics (such as frequency and field strength) and the method applied will or may advantageously be customized to suit the particular patient and/or the condition to be treated for a patient, or set according to both the particular patient and the condition to be treated for the particular patient. Furthermore, as treatment progresses over time, the treatment characteristics may be altered either according to predetermined plan, treatment regime, or policy or in response to feedback from the patient. However, it will be appreciated in light of the description provided here that a therapeutically effective treatment may be provided by a set of default device characteristics within the defined ranges even if those device characteristics do not provide a maximized or optimized treatment or therapeutic benefit for all patients.

When a fifty-percent (50 %) signal duty cycle (half "ON" time and half "OFF" time), the frequency range of 15 Hz to 60 Hz maps according to the relationship frequency (f) equals $1/(2 \times t1)$ into the waveform equation having the time parameter t and time constant parameter $\tau$ as follows:

$$I(t) = \begin{cases} 0 & , \ t < 0 \\ I_{max} \times (1 - (1/e^{t/\tau})) & , \ 0 \leq t \leq t_1 \\ 0 & , \ t > t_1 \end{cases} \qquad \text{Equation (3)}$$

While the fifty-percent duty cycle has been found to be effective, operation of the device, system, and method are not limited to a fifty-percent duty cycle, or even substantially fifty percent duty cycle (e.g., with a 10% variation). where

$$8.3 \ \text{msec} \leq t_1 \leq 33.3 \ \text{msec}$$
$$1.25 \text{msec} \leq \tau \leq 2.0 \ \text{msec}$$

The range of values for other frequency ranges may be calculated according to the expression: frequency = $1/(2 \times t1)$ for a fifty-percent duty cycle.

In addition to the frequency and the field strength, the point of application of the focused electromagnetic field is also critical to the treatment efficacy. Through testing, it was determined that there are two plausible points of application for the treatment to be effective. One point is in the center of the patient's palm and the other point is the center of the sole of the patients foot. At both the palm of the hand and the sole of the foot, a heartbeat or pulse may be detected by the heartbeat sensors described herein. Also, both of these locations have an acupuncture point, at acupuncture point in the palm area of the hand (at acupuncture point PC8 or LaoGong) and at the acupuncture point at K11 or YongQuan) on or near the sole of the foot.

In an embodiment of the present invention, the cell or tissue excitation terminal 5 may be used as a separate component to operate, and the optional server 1 is not needed. In this case, the processor, processing logic circuit, or CPU 50 can control the whole device 5 to perform therapy on a patient, meanwhile the database 11 of the server 1 and other functional modules are integrated into the cell excitation terminal 5 or the processor or CPU 50, and when the cell excitation terminal 5 works alone, and the cell or tissue excitation terminal 5 may be provided with generation, storage, and downloading of new and modified encoded data waveforms (which therefore may be applied to the patient) anytime and anywhere through a cell phone network or other wired or wireless communication network, thereby also achieving the objective of the present invention.

It may also be appreciated that only an intermittent connection or coupling with an external information and/or processing source such as the server 1 and/or database 11 may be needed.

An example working process 1100 of the therapeutic system according to a non-limiting example of the present invention is described below.

First, the cell excitation terminal 5 is physically located, placed, or fixed on a part of a human body (Step 1101). For example, at or near an acupuncture point of a body part such held or otherwise attached to the palm of a human hand, stepped upon or attached to the sole of a human foot, at an arm, or at another location. The cell or tissue excitation terminal 5 is very small (in one example, generally between about 7.62 cm wide x 10.16 cm long x 5.08 cm long x 2.54 cm high or thick (3 inches wide x 4 inches long x 2 inches high or thick) as it would extend from the body when applied and 2.54 cm wide x 5.08 cm log x 2.54 cm high (1 inch wide x 2 inches long x 1 inch high)) and therefore is very easy to be fixed or located on a body part.

Optionally but advantageously the cell or tissue excitation terminal 5 may be provided with some attachment means, such as straps, a renewable adhesive surface, taped, or simply placed on the body region to receive the treatment. In one non-limiting example, the excitation terminal may be held in the hand or palm of the hand with the housing over the emitter in contact with the palm of the hand so that it may readily be held in place by the person undergoing treatment. Alternatively, Velcro straps may be used to attach the excitation terminal device 5 to the hand. In yet another non-limiting example, the excitation terminal device 5 may be placed in a pocket in a glove worn on the hand of the person receiving treatment, the glove optionally having a hole or aperture that permits the emitter surface to be in contact with the palm of the hand. When the excitation terminal device 5 is adapted for treatment at the palm of the hand, a sensor that uses an Infrared (IR) beam may be used to measure patient status information at a fingertip, such as blood flow, oxygenation, pulse, heartbeat, and other parameters. Furthermore, a skin conductivity surface may also be provided that monitors and measures moisture on the patient's skin as this can be an indicator of mental or emotional stress or other physiological condition and may provide useful information as part of the customization of waveforms.

The cell or tissue excitation terminal 5 is then powered on (Step 1102) such as by moving the position of a physical on-off switch or by sending a command or signal via a wired or wireless communications link. In this case, once the cell excitation terminal 5 starts working, the heartbeat sensor 58 detect human body status or subject information constantly or at predetermined or dynamically determined sampling intervals (Step 1103), and sends or communicates the human body status information to the processor or CPU 50 (Step 1104).

Then, when an external server 1 and database 11 are configured, the human body status information is sent (Step 1105) to the database of the remote server 1 through the communications link or network, such as to a private or public wired and/or wireless network to be stored (Step 1106) and later optionally accessed (Step 1107) for future reference, dynamic and adaptive customization, and other operations and uses as described elsewhere herein.

According to the human body status information and stored or timely measured other physical status parameter data, a corresponding bioelectromagnetic wave (information) and therapeutic manner is selected (Step 1108) for the patient from the mapping relationship list or table of patient sickness and waveforms. The processor or CPU 50 of the cell or tissue excitation terminal 5 sends data to the D/A converter, controls (Step 1109) the waveform multiplexer 56 (if present), the driver 561 (and driver 562 if present), and the waveform generator 581 (and waveform generator 582 if present) connected to the driver 561 (and driver 562 if present) to generate (Step 1110) the corresponding bioelectromagnetic wave and use the therapeutic method to perform therapy on the patient directly.

In examples having only a single waveform generator, the processor or CPU 50 of the cell or tissue excitation terminal 5 sends data to the D/A converter, controls (Step 1109) the he driver 561, and the waveform generator 581, connected to the driver 561 to generate (Step 1110) the corresponding bioelectromagnetic wave and uses the therapeutic method to perform therapy on the patient directly.

In addition, optionally but advantageously during the therapeutic process, the sensors in the cell or tissue excitation terminal 5 further detects (Step 1111) the human body status information of the patient constantly, and transfers the information (Step 1112) to the processor or CPU 50 and (when present) to the remote server timely (Step 1113), so as to timely adjust, select, or change (Step 1113) the bioelectromagnetic wave for radiation therapy. This effectively completes the therapy or therapy session at least for a period of time.

In addition, optionally but advantageously after the therapy is completed, the EM wave, the therapeutic means, and a therapeutic effect (which can be reflected by or analyzed from the detected human body status information or other test, status, or subject information) of the therapy are sent (Step 1114) to the server 1 to be stored (Step 1115).

After the number of the patients reaches a certain value or even an individual patient has received several treatments, the optimization module of the remote server may update (Step 1116) the mapping relationship list of patient sickness and waveforms with the waveform and therapeutic means corresponding to, for example, the a well performing effective or "excellent" therapeutic effect among the therapeutic effects. In this way, the patient may receive EM radiation updated settings and waveforms from time-to-time, thereby providing and in many instances guaranteeing constant dynamic and adaptive optimization of the therapeutic effect, and meeting requirements from ever changing physical status of the human body.

In addition, the therapeutic system according to the present invention may optionally but advantageously be controlled remotely, so that the therapeutic EM waves may be adjusted or changed during the therapeutic process of the patient, so as to be adapted for various changes of the human body in a timely manner. In some non-limiting treatment regimes, there may even be prescribed changes over a period of time, such as applying a first type or characteristic during a first period of a treatment session and then applying a second (or subsequent) type or characteristic during a second (or subsequent) period of a treatment session.

In an embodiment of the present invention, timely dynamic and or adaptive change and/or customization of the therapeutic EM wave may further improve the therapeutic effect. Non-limiting examples are further described below.

In one further specific but non-limiting example, (1) the EM wave therapeutic method being used may also vary according to different situations. For example, by sensing a heartbeat or pulse frequency of a patient, the heartbeat frequency of the patient is used to directly synchronize or change a specific EM pulse or a set or series of EM pulses within a period of time before, during, and/or after a heartbeat or local blood pressure pulse, or alternatively the sensed heartbeat frequency is used to determine when and where changing or replacement is performed by using a different or modified EM pulse or EM pulse set. It may be appreciated that since often the heartbeat or pulse rate and expected time of the next heartbeat or local pulse does not vary too much from beat to beat or pulse to pulse during a few seconds of time, the synchronization may be accomplished either by sending the heartbeat or pulse, by predicting the heartbeat or pulse over a short period of time, by a combination or these two, and/or in other ways.

For example, in a non-limiting example, a selected first wave and specific waveform properties of that selected first waveform may be used to change the EM wave by a certain manner, for example, by changing a waveform shape and/or frequency components, an amplitude, a combination of amplitude and waveform shape or frequency components, timing, or other properties. These changes may be made, for example but not by way of limitation, between heartbeats or between blood pressure pulses but at time corresponding to the heartbeats or local blood pressure pulses at the area or region of the patient body where the EM wave therapeutic treatment is being applied, which may be a change applied to only modify the EM pulse at a rate being 30 Hz (30 times or cycles per second) corresponding to or synchronized with the heartbeats (for example, 60 to 120 heartbeats per minute), or may be used to preset or dynamically determine the number of the EM pulses, or preset or dynamically determine a time period at or approximately at the heartbeat or the blood pressure pulse.

In another further specific but non-limiting example, (2) a device is used to modify an EMF (magnetic field and electric field), or modify only a magnetic field, or modify only an electric field based on timely human body status information of a patient. For example, a response to an EMF (for example a heartbeat frequency or a heart rhythm change), so as to achieve a desired effect, thereby further improving the therapeutic effect. For example, heartbeats, the heat rhythm, and/or, for example, other heart-related patterns or conditions sensed or acquired from an instrument such as an EEG and/or ECG, are used as feedback to the system according to the present invention. Optionally, an extra sensor device is disposed in portable equipment or separately to sense other body symptoms of a patient. For example, an EEG signal may be acquired from a human body, particularly from a head area of a patient or a person undergoing therapy. Multiple sensors or sensing devices may be used at different locations at or near the patient body or tissue being treated

According to a non-limiting example or the present invention, the EM wave generators are set by configuring the generators to be able to use a relatively low-frequency pulsed magnetic field to stimulate blood circulation in a body of a patient. The low-frequency normally ranges from 10 Hz to 200 Hz, typically ranges from 20 Hz to 120 Hz, more typically ranges from 20 Hz to 40 Hz, and in one particular implementation is about or actually 30 Hz (for example a frequency with up to about 10-percent variation, $30\pm3$ Hz) in a specific case.

In addition, in non-limiting examples, the present invention may be applied as a diagnostic tool or instrument and not directly for treatment, or it may be used for diagnose and later or concurrent treatment. In one example, the present invention can enable a therapist, for example medical personnel (or persons in charge of therapy on themselves or others) to modify the waveform and frequency of the magnetic field and the shape and amplitude of the waveform to be adapted for diagnosis of a patient. Various waveforms may also be applied alternately, and waveforms applied to a part of the body or meridians of the patient may be different from waveforms applied to a different part of the body or meridians of the patient.

In addition, when multiple sets of portable equipment are available, the multiple sets of portable equipment can use the same or more typically different waveforms and waveforms capable of being dynamically modified, which are applied to different parts or different meridians of the human body at the same time. Furthermore, multiple sets of devices or other equipment as described herein can be used together with other equipment phased according to a manner, so that only one set or a selected subset of all of the equipment can operate (emit electromagnet field) at the same time. Therefore, multiple sets of equipment can be applied to different meridians of a single patient and is controlled according to a programmed therapeutic scheme (for example through a timer or local wireless or wired connection) to operate. In this manner a patient may receive or have applied multiple therapeutic treatments to different body parts and/or using different EM wave characteristics, or both in an efficient manner without requiring continuous manual changes by a therapist or therapy technician. For example, several different cell or tissue treatment devices 5 (for example 6 such devices) may be located or fixed on different portions of a single patient's body and they may be automatically programmed and controlled so that during a first period of time two of the devices emit the same EM waves over different portions of the body, then the two devices are turned off or stopped from emitting and one other device operates, then a period of time after that two other devise at still other points on the body are operated using either pre-programmed EM waveform characteristics that were known in advance or that were determined during the operation and sensing of the other devices.

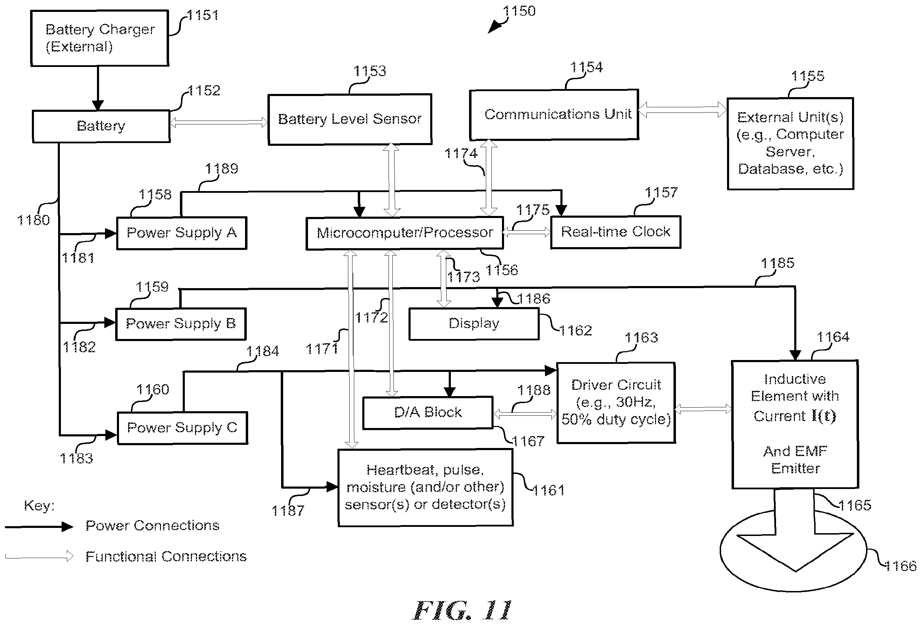

FIG. 11 is a further block diagram of another example of the inventive excitation device showing a communications unit that may be used to connect to an external computer, information appliance, smart-phone having computing and data storage capability, or computer server, such as server 1 and/or database 11 as already described.

Microprocessor, processor, processing logic, or central processing Unit (CPU) 50 provides operating control over the various internal circuits and components and also generates a digital signal such as a signal in the form of a pulse width modulated digital pulse train that is communicated to and received by a smoothing element that converts the digital stream to a smoother (and generally lower frequency) analog signal. Examples of the device 5 may use a low-pass filter, other filter, and/or a digital-to-analog signal (as shown) to generate the signal sent to the Driver module. Storage for digital information such as data, software algorithms, control, patient data, and the like may be stored in a memory within the processor or in a separate memory coupled to the processor. An optional memory card slot for receiving a removable memory card, such as a flash memory card, microSD memory card, or other memory cards as are known in the art may be provided and exposed on the outer surface of the exciter terminal. Optionally a USB port can be provided for loading, storing, or reading data, software, or other information. These may be in addition to any wireless interface or communication means provided.

A real-time clock is advantageously provided so that treatment or therapy time may be accurately controlled and so that patient status information that is sent to the database and used for optimization may have an accurate time stamp and date stamp.

The Display may be any convenient display such as an LCD, LED or other display for presenting information to the therapist or patient before, during, or after use; providing a device 5 self diagnostic check, and/or other information.

The Digital-to Analog Block or module is based on a Pulse Width Modulated (PWM) output such as a pulse train with a time varying duty factor, from the processor is received. The PWM pulse train is then processed by Combining PWM and a low-pass filter that forms a cost effective digital-to-analog converter, providing an accurate and simple wave shaping and synthesis function. A simple and effective low-pass filter can be formed by using an resistor-capacitor pair. Waveforms of different shapes, frequencies, and amplitude may be generated by combining such a PWM system with a low-pass filter. More advanced D/A convertors can also be used in the terminal to perform even higher accuracy waveform synthesis.

The Drive Block circuit or module is operative to generate the drive signal to the inductor element, such as the signal I(t) as described.

The Inductive element includes an electrical inductor typically in the form of a coil of wire and some resistance R resulting from the wire itself. The inductive element may be modeled as an ideal inductor in series with an ideal resistor and acts as a load for the signal received as an output from the driver.

Sensor(s) or detector(s) such as a heartbeat rate detector, a pulse rate detector, a blood oxygenation detector, a skin moisture detector, a skin or body temperature sensor, and or other detectors or sensors may be used alone or in combination to provide patient physiological status information that is used and optionally but desirably stored for each treatment so that the waveforms may be dynamically and adaptively changes or modified so that therapeutic treatment may be optimized.

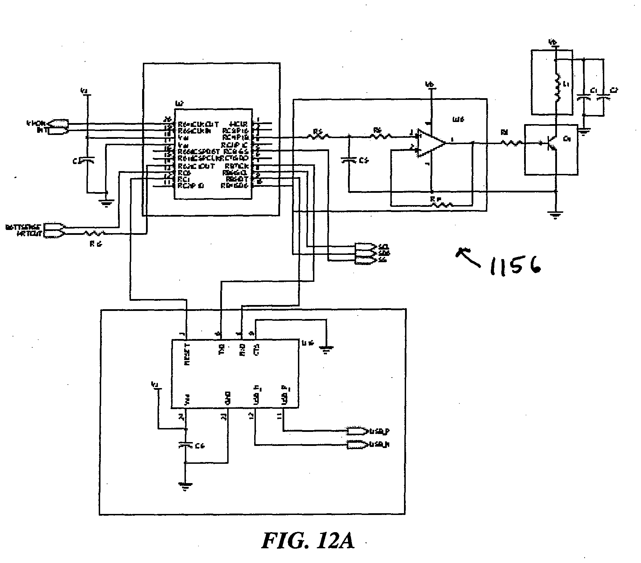

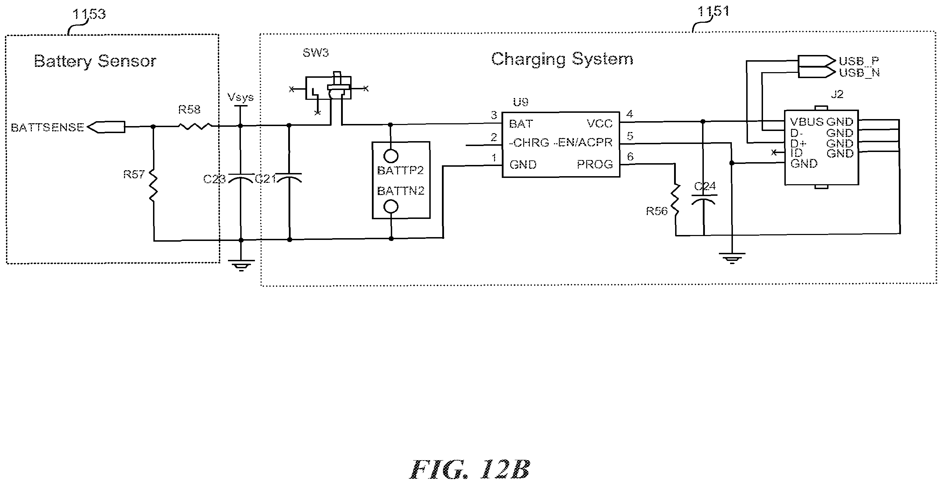

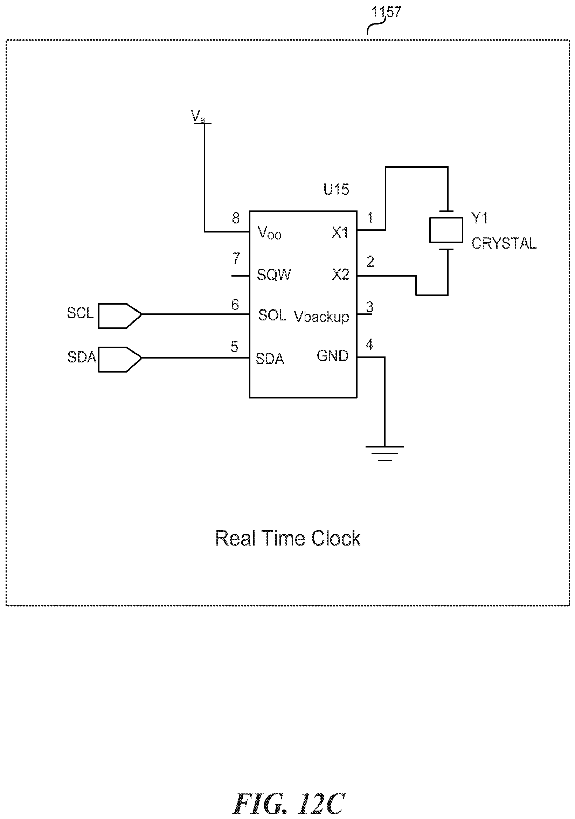

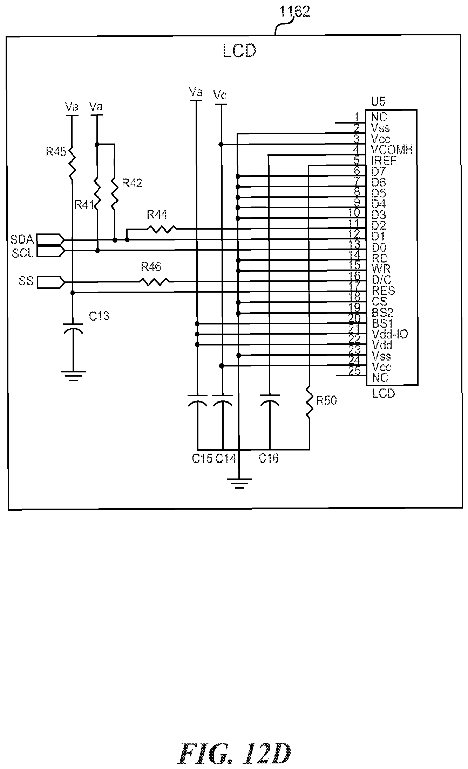

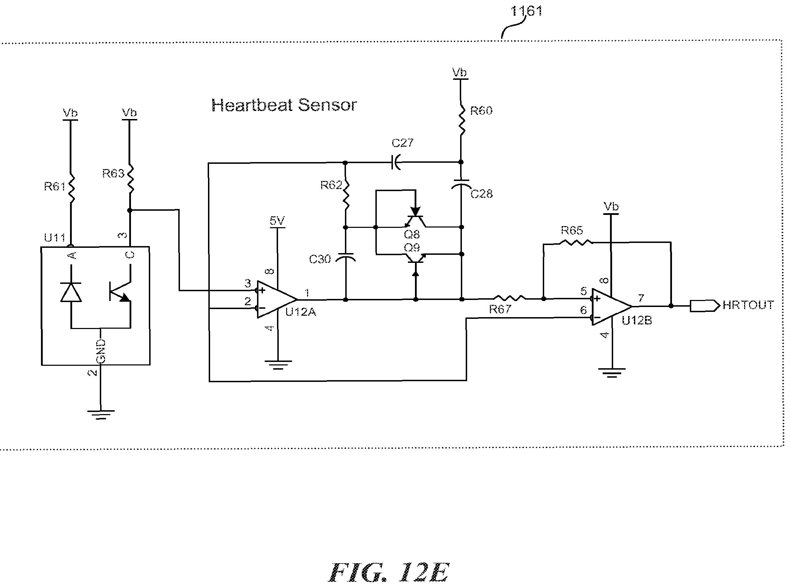

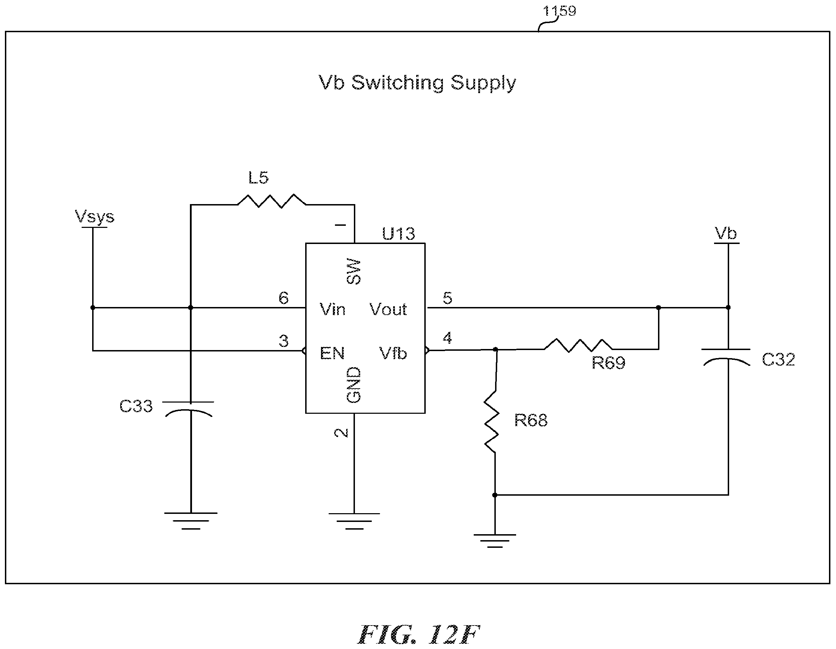

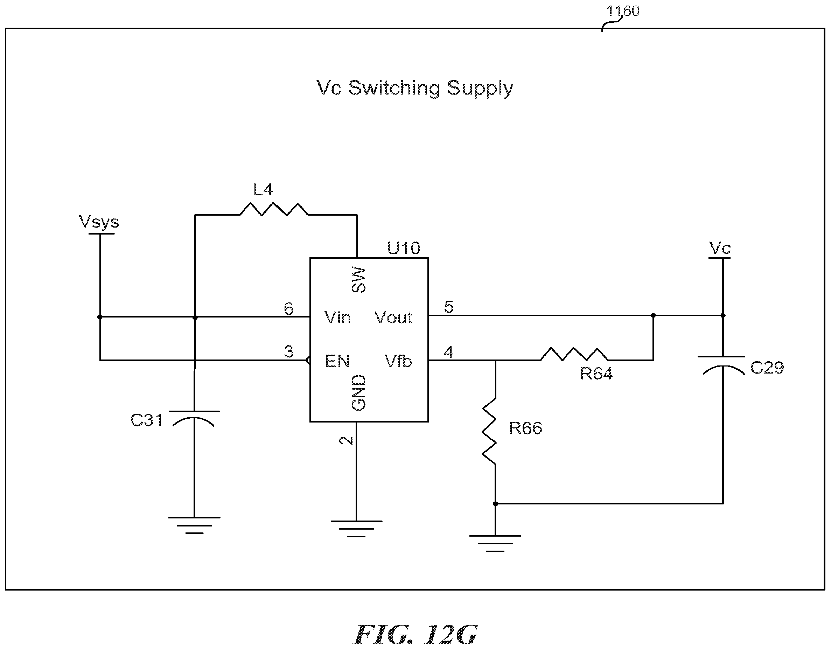

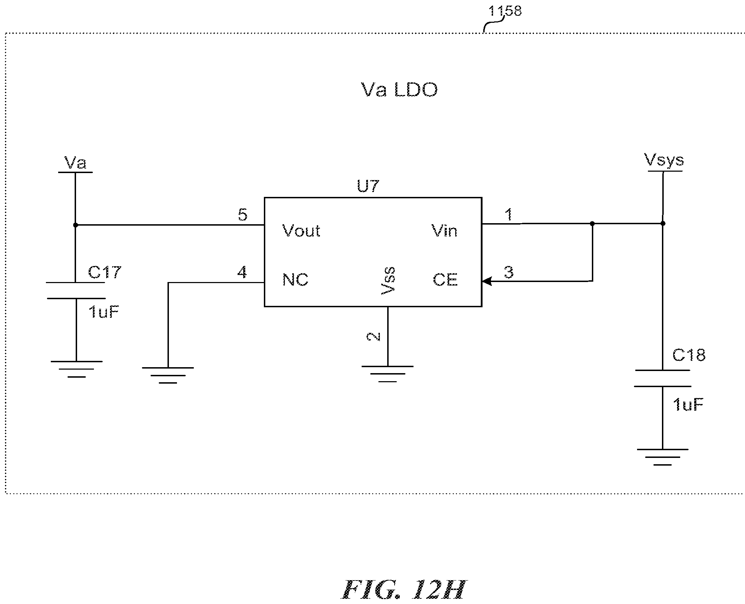

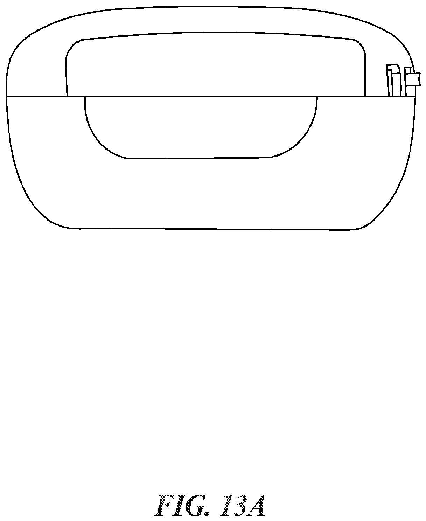

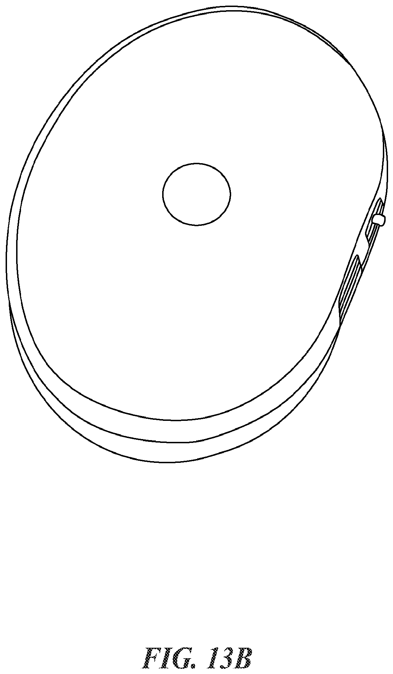

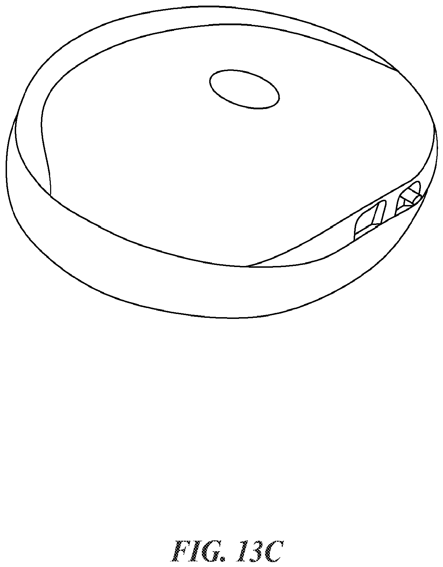

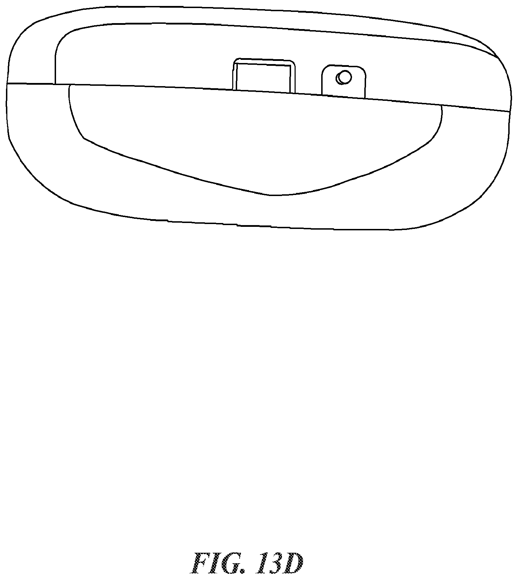

With reference to FIG. 12, there is illustrated additional detail for the functional blocks. FIG. 13A is an illustration showing additional detail for the Microprocessor block or module. FIG. 13B is an illustration showing additional detail for the Charging System and Battery Sensor blocks or modules. FIG. 13C is an illustration showing additional detail for the Real Time Clock block or module. FIG. 13D is an illustration showing additional detail for the Display or LCD block or module. FIG. 13E is an illustration showing additional detail for the Heartbeat Sensor block or module. FIG. 13F is an illustration showing additional detail for the Vb Switching Supply block or module. FIG. 13G is an illustration showing additional detail for the Vc Switching Supply block or module. FIG. 13H is an illustration showing additional detail for the Va LDO block or module.

It may be appreciated that since heartbeat rate is an important human physiological parameter (indeed a vital sign), that it serves additional purposes beyond the dynamic waveform and treatment modification. In particular, it further provides a patient safety feature so that of the heartbeat rate is out of an acceptable range before treatment begins, the processor may inhibit operation of the excitation unit, and if the heartbeat rate goes out of an acceptable range during a therapeutic session the session may be terminated and depending on the heartbeat range a signal or communication may be sent to the therapist or to a medical professional at a remote location so that the reason for the out of range heartbeat may be identified and help summoned if necessary.

The three power supplies or sources provide operating voltage and current for the different circuits and modules within the excitation terminal device 5.

With reference to FIG. 13, in which FIG. 13A - FIG. 13D are illustrations showing the physical configuration and small size of an example of the portable excitation terminal treatment device. One example has dimensions of substantially 6.99 cm x 7.62 cm x 2.54 cm (2.75 inches x 3 inches x 1 inch) while a different example is dimensions of substantially 6.6 cm x 5.84 cm x 2.54 cm (2.6 inches x 2.3 inches x 1 inch).

It is anticipated that some further reductions in size may be made in any final product; however, the intention is that the portable device have a size that permits application to the body part or meridian without undue effort or discomfort, and that the portable device be small enough that it may be readily transported in a pocket or purse if desired. Various buttons and switches and sensor apertures are shown on the device as well as a charging connector for maintaining battery charge and/or recharging the internal battery. FIG. 12A shows an end view, FIG. 12B shows a first perspective view from above, FIG. 12C shows a second profile view from the side, FIG. 12D shows a side view of the example portable device.

In another example of the portable excitation terminal or device 5, the case or housing is adapted to be placed or held in the hand and useful for treatment at the acupuncture point in the palm of the hand (Also, both of these locations have an acupuncture point, at acupuncture point in the palm area of the hand (at acupuncture point PC8 or LaoGong) and at the acupuncture point at K11 or YongQuan) on or near the sole of the foot.) though not limited for that purpose. In this example of a portable excitation terminal device adapted for holding in the palm of the Hand (at or near the acupuncture point at LaoGong). A display of the Heartbeat rate or other information is provided. The display may also be programmed to cycle through different status information or have an input that permits the user to school through or otherwise determine an operating or status condition. The shape of the housing is adapted to center the EM wave emitter area at the optimum point or region for treatment. Furthermore, one or more sensors for measuring heartbeat, skin moisture, pulse, body temperature, room temperature, or other sensed status or information are advantageously disposed on a suitable location on the housing so that intended sensed information may be obtained.

It may be appreciated that this particular example provides an ergonometric size and shape for placing or removable attachment to or at the palm of the human hand. Different sized housing may be provided having the same or substantially the same internal electronics so that the device 5 may fid different hand sizes (such as small, medium, or large hands, and place the emitter at an optimized location within the palm of the hand and when provided place the IR sensor port or aperture to optimally match the location of a finger or finger-tip portion for sensing of heartbeat, pulse rate, blood oxygenation, and/or other status information or physiological data on the patient before, during, and/or after treatment. An electrical conductivity sensor may also be provided on a surface of the housing in a location that will contact the skin of the hand. A display is also advantageously provided to provide user instructions and/or information and data either automatically or response to an internal or external signal or query.

**Additional Description**

In one aspect, an example of the small (generally about the size of a handheld cell phone or PDA device) portable battery powered device provides several features and advantages as compared to conventional devices, systems, and methods. By way of example, but not of limitation, these features include:

(1) Physiological and/or patient monitoring sensor(s) or detector(s) or other means for sensing the patients' heartbeat or pulse frequency and synchronizing or altering a particular electromagnetic waveform pulse or set of pulses during a period of time immediately before, during, and/or immediately after the heartbeat or local blood pressure pulse has passed a region with the patients' heartbeat frequency or rate, or alternatively using the sensed heartbeat frequency or rate to determine when in time to alter or substitute a different or modified waveform or waveform characteristic during a single therapeutic treatment session or from treatment session to treatment session. For example in one non-limiting example, a chosen first waveform and its particular waveform attributes may be used between heartbeats or blood pressure pulses but at a time corresponding with the heartbeat or local blood pressure pulse altering the electromagnetic wave in some manner, such as by changing the waveform shape and/or frequency components, amplitude, or other characteristics. This may be a change for a single modified electromagnetic pulse that is applied at a 30 Hz rate (30 times per second) corresponding to or synchronized with the heartbeat (for example, at a heartbeat at 60 to 120 beats per minute), or may be for a predetermined or dynamically determined number of EM pulses or predetermined or dynamically determined duration of time at or around the time of the heartbeat or blood pressure pulse. Additionally, the sensor(s) or detector(s) and/or other means for sensing such as the blood oxygenation sensor, skin moisture sensor, skin temperature sensor, or other sensors alone or in any combination may be used to determine a change or modification in waveform, and/or determine when such change or modification should be made. These changes or modifications may be made relative to heartbeat or pulse rates and occurrence, or independent of such timing or occurrence.

(2) Waveform generation circuits or other waveform control, selection, shaping, or other measures for generating and/or modifying the electro-magnetic field, or only the magnetic field, or only the electric field to achieve the desired result based on the patient's reaction to the electro-magnetic field (such as based on a sensed heartbeat frequency or rhythm change or other sensed or detected patient physiological condition). It may be appreciated that various heartbeat, heart rhythm, and/or other cardiac related patterns such as may conventionally be obtained from a heartbeat sensor, blood pressure sensor, electrocardiogram, EKG, and or other sensors alone or in any combination may be used as a feedback to the portable excitation terminal or device. Alternatively, or in addition, sensor(s) or detector(s) or other sensing means may be provided within the portable device or separately external to the device to sense other patient conditions, such as brainwave patterns that may be sensed from the body, particularly from the head region of the patient or person undergoing the treatment. Such sensed signals from external devices may be communicated to the portable excitation terminal or device 5 via a wired or wireless communication link (such as via a Bluetooth or WiFi communication link), or communicated to the server 1 via a communications link for substantially concurrent use with the operation of the computer server, portable device combination, or stored for subsequent use during a period of analysis for later waveform selection and optimization.

(3) The electromagnetic wave generator is configured to radiate a low-frequency, focused or diffuse EM field with different degrees of focusing or emission patterns possible, pulsed magnetic field(s), where such low frequency may usually be between about 10 Hz and 200 Hz, more typically between 20 Hz and 120 Hz, even more typically between 20 Hz and 40 Hz, and in one particular example about or substantially 30 Hz (e.g. 30Hz $\pm$ 3 Hz). These low-frequency EM pulses (substantially 30 Hz $\pm$ 10%) have in particular been found to have a beneficial therapeutic effect to stimulate blood circulation in a patient's body.

(4) To use the same low frequency, pulsed magnetic field to stimulate and/or improve the user's circulation and to re-animate the person's potentially aged and dormant blood cells. It has been found experimentally, that this form of therapeutic treatment has been effective in reducing the formation or presence of blood cell rouleau or rouleaux which are generally linear stacks or clumps of red blood cells thought to form because of poor health and inflammation of something in the patient's system and the disc-like shape of the red blood cells in vertebrate bodies such as in human patient bodies that permit such clumping when there is a poor health condition.

(5) To provide treatment personnel, such as medical personnel (or the person if self-administering the treatment), with the ability to tailor or customize the waveform frequency, waveform shape, and waveform amplitude or characteristics of the electromagnetic field and particularly the magnetic field component of the electromagnetic field to the diagnosed sickness or condition of the patient. It may be appreciated in light of the description provided herein, that the analytic and computational features described elsewhere herein (such as the server, database, processors or CPU) may provide the customized waveforms for the treatment and perform the dynamic adaptation or modification, and that the user need not be responsible for making the waveform changes or selections directly. Multiple different waveforms may also or alternatively be applied and the waveforms applied to one part of the person's body (such as to the palm of the hand, sole of the foot, acupuncture meridian lines or points, or at other locations) may be different than that applied to a different body portion. Furthermore, when multiple portable devices are available, they may be simultaneously applied to different portions of the body using either the same or more typically different waveforms and dynamic modification. Yet still, multiple devices may be applied in a constellation with the different portable excitation terminals or devices being phased as a function of time in some way so that only one or alternatively a selected subset that is all or less than all of all the devices operate (that is, emit electromagnetic or EM field) at the same time. Thus, multiple portable devices may be applied to a single person's body at different body locations and controlled (such as through timers, real-time clocks, or a local wireless or wired connection) to operate according to a programmed or dynamically determined treatment pattern or regime.

(6) Generation, storage, and communication or data upload and download means for providing new and tailored or customized encoded digital waveform(s) to the portable excitation terminal or device 5 (and hence available for therapeutic application to a patient) anytime and anyplace through the use of an available communications link, system, or other communications means. In one non-limiting example, the cellular phone network or other wired or wireless communication network may be used, and when the patient will operate the portable excitation terminal or device 5, he or she may effectively use their own cellular telephone to provide the means for uploading and downloading status information and/or waveform signal characteristics (such as encoded digital waveforms) or an identifier of such encoded waveforms if they are already stored or available in the portable device or stored on media in possession of the user or therapist. It is noted that the portable excitation terminal or device may be made with a particular set of waveforms and/or programmable or selection features so that it may be used without any other external devices, network, or external server. In some instances, a doctor or healthcare provider may preprogram the waveforms in after the patient's visit and also program or set the acceptable range of physiological parameters at the time. In particular such portable device may be made and used without the server, patient cell phone, or other external system. Such a self-contained portable device may include a more powerful processing device, include additional solid state memory for storage of user data and waveforms, may include means for loading new information or waveforms from an external source (such as from a memory card or USB or other communications port) even though it may not rely on such external source during operation. The customization of waveform dynamically and adaptively during a single treatment session or from different session to different session may then rely on the internal processor or CPU, sensors within or in local communication with the excitation terminal or device and collecting the sensed physiological data, and then use this to autonomously modify or change the waveforms. In one non-limiting example, the self-contained excitation terminal or device may also access an external server or database to obtain updated waveforms, send user's data for more rigorous analysis and/or comparison with other different patient's information, and the like; however, not rely on such external system server or databases for routine treatment. The safety features provided by the heartbeat sensor or other sensors or detectors may still be enabled to inhibit EM field emission in the event that a physiological status or parameter is out of an acceptable range. In this situation, the patent may have to obtain some further consultation before the excitation device may be operated. Provision of some ability to change some thresholds for operation may be made available where there is a known and understood reason for an otherwise out of range physiological parameter.

(7) Provide the electromagnetic radiation at the one or more body locations (such as the human hand or foot areas or at or along acupuncture meridian points or lines) at sufficient intensity without the potentially unknown effect of bathing the entire patient body with a larger total or integrated EM field, even where the intensity or field strength applied to the body part is lower or comparable for the portable device.

(8) Provide an ability to shape the electromagnetic wave emitted from the portable device so as to have the desired spatial treatment pattern and intensity within the body or body part while minimizing power consumption by the device and maintaining sufficient beneficial treatment effect.

It may be appreciated in light of the features described above and elsewhere in this patent that unlike the conventional devices that take advantage of electromagnetic fields for a claimed therapeutic usage, the present inventive system, device, and methods are different and much more advanced in numerous ways that improves on efficacy of treatment:

For example, the inventive system and portable device and treatment method provide for dynamic and customized pulsed electromagnetic fields as compared to conventional systems and devices that provide only a static magnetic field or where the waveform may vary over time, such waveform is repeated and not dynamically modifiable or adjustable in response to sensing of the patient's body.

More particularly, there may be systems or devices that are magnet-based (these may be fixed permanent magnets made from a solid piece of magnetic metal or other magnetized material with constant magnetic field strength) and the user or patient just applies the permanent magnet to any local area (i.e., wrist band, necklace, or the like). There may also be metallic devices to be worn on the body that are claimed by others to focus or concentrate ambient electromagnetic waves or magnetic fields from the environment for beneficial treatment effect. There may also be somewhat portable units where a permanent magnet is mounted on a motor and the rotated to modify the magnetic field because of the rotation, but again there is no way to modify the rotational behavior of such devices after they are made and no way to customize the waveform to customize it to the patient's condition, physiological characteristics or status during treatment, or to the other advantages and features of the present invention.

There may also be large non-portable clinical systems where the electromagnetic field is pulsed, but the only control that the user has is the field strength (i.e., amplitude) while there is no intelligence in the system to monitor or sense the patient's status (i.e., heart rate changes, pulse rate changes, blood oxygenation, skin moisture content, body temperature, alone or in any combination) during treatment and dynamically compensate (such as modifying the waveform shape, frequency characteristics, amplitude, pulse rate, or to choose and apply a completely different waveform shape, in real-time, substantially real-time with some slight communications delay, or from treatment session to treatment session) to achieve the optimized results. Timing circuits or processor algorithms, and/or other waveform transition means may also be optionally provided to transition between different waveform characteristics and/or waveforms to as to gradually alter the effect on the patient and to minimize any stress, shock or possible negative effects from fast turn on or turn off and/or fast switching from one waveform shape and/or characteristic to another waveform shape and/or characteristic. In some non-limiting examples, this transition means may include the processor or CPU 50 within the excitation terminal or device 5.

Also, it is known that different patients will react to different electromagnetic wave patterns so in the inventive portable excitation terminal or device and system, the wave shape pattern or waveform and waveform characteristics are dynamically or programmatically changeable for the best therapeutic effect and efficacy.

As earlier described, the inventive device and system provide for selective application of electromagnetic fields onto parts of the human or other animal body (such as to the patient's hand or foot, along blood vessels exposed near the patient's skin, and/or at or along meridian points or lines) on the body using known controlled field strength and dosage, as compared to conventional systems that involve bathing the patient's entire body in an overall magnetic field. Recall that in one non-limiting example, the inductor element that includes a wire coil emits from an area that is between about 1.27 cm (½ inch) to about 5.08 cm (2 inches), and in one particular example, the inductive element coil emits over an area that is substantially 2.54 cm $\pm$ 0.51 cm (1 inch $\pm$ 0.2 inches) which is much less than the entire human body.

These conventional systems, devices, treatment methods, and platforms are indiscriminant in the electromagnetic field application location. They either bathe the entire body of the user in the electromagnetic field, or it asks the patient to self-locate the location of treatment to where they are uncomfortable. They do not include any sensing means for assisting the user or the patent to locate the optimum region of application, whether by sensor feedback, or in other ways. It will also be appreciated that the size and ergonometric shape of the housing of at least one example of the inventive portable excitation device provides a size and shape that provide a functional advantage in that it assists in locating the location where the EM waves are emitted at the correct location into the body, such as in the center of the palm of the hand. For example, it has been determined that in addition to beneficial effects when applied to the palm of the hand or sole of the foot, there may be beneficial effects from treatment at a meridian point closest to or proximate the location of the patient's pain or discomfort. While patient self location of the treatment point might work in some instances based on luck, the efficacy is uncertain, treatment sessions that do not have appropriate and suitable electromagnetic or magnetic characteristics may be wasted.

It may therefore be appreciated in light of this description, that using the body meridian system developed by Chinese physicians (used for hundreds of years in different fields of Chinese medicine, including acupuncture), the inventive portable device alone, and more especially when used in conjunction with the system including with the external server, its processing and analytics as well as with the database with its multiple patient sickness and treatment records, is able to target specific sickness, conditions and bodily functions. It may be appreciated that the supporting system, provides a means and mechanism for storing and loading data to or from the portable device, generating waveforms, and other higher level functions; however, it is the portable device that actually generates the electromagnetic waves for treatment. One advantage of the use of the supporting system and server and the wireless cell phone connection (or other communication link or system) is that it allows the portable device to be made less expensively and to sell at a lower price at least in part because the purchaser already has s cellular telephone. It also permits the device to operate on lower power and in some instances to be smaller in size. Furthermore, in some instances, the electromagnetic wave generation circuits may interfere with certain processor or logic circuits if they were physically in the same device as used for long range communication to a server, so there are technical advantages gained from somewhat physically separating the communication and processing logics from the actual electromagnetic wave generation circuits and emitters in the portable device. In some non-limiting examples, the communication, such as via local Bluetooth wireless signal may take place between electromagnetic emission or the emission may be paused for very short coordinated periods for data exchange to occur.

The inventive portable excitation terminal or device (alone or in conjunction with the other components of the system) also provide dynamic, targeted waveforms tailored and customized to the patient body characteristics and condition to be treated. Conventional systems have used the same waveform and waveform characteristics for everyone, at most changing only the fixed waveform's amplitude.

Conventional devices and systems on the market support different electromagnetic field strengths/amplitudes (but only use the same waveform if it is a pulsed system), and advocates of such same single waveform systems and devices suggest that such single waveform is universal and sufficient to cover different patient types, patient conditions, and patient use cases.

By comparison, the present inventive portable excitation terminal or device and system use different waveforms and different waveform characteristics (with an ability to dynamically modify the waveform shape, type, frequency, pulse, and/or amplitude characteristic) either programmatically during a treatment session or dynamically using sensed feedback from the patent collected by on-board sensors during treatment, in order to maximize the efficacy for the particular patient among different users (and to target different functions of the body).

Therefore, aspects of the invention are directed to providing means and method for changing more than just amplitude or field strength, and that each person has a different tolerance and susceptibility to electromagnetic fields, and may typically benefit differently from different waveform types and characteristics beyond mere amplitude or field strength changes.

Moreover, in the inventive portable excitation terminal or device and treatment device and system, means and method for sensing or monitoring the patient's heartbeat, and/or other physiologic characteristics ,in order to: (a) synchronize one or more particular pulse radiation waveforms that may differ from the pulses emitted between heartbeats at the point of treatment with the heartbeat if desired, and/or (c) to monitor the patient's reaction to the process to fine tune or customize the waveform type or characteristics. Recall that the use of a digital pulse train and then to filter or digital-to-analog convert that pulse train to generate an inductor element drive signal gives both a broad ability to generate different wave types and to fine tune with precision the generated EM waves whether pulsed or not pulsed hat are emitted.

In those treatment examples here there is a synchronization of the emitted EM wave pulses with the heartbeat, the synchronization described is not to synchronize the frequency of the emitted EM pulse (for example at about 30 Hz) with the heartbeat (at about 60 beats per minute, or about 1 beat per second or 1 Hz) in terms of them being at the same frequency, as the typical heartbeat falls in a range of 50 to 120 beats per minute and the range of frequency for the EM pulse emission is on the order of 20 to 100 Hz (times per second) and in one particular example is substantially 30 Hz.

The synchronization described refers to altering the waveform and/or its characteristics at a time corresponding to the heartbeat (or to the detection of the local blood pressure pulse or change) at the treatment site. Without benefit of theory, this change may be beneficial as it is a short period of time at which the body may derive particular benefit from specialized of different pulse characteristics. Matching one or a small set of EM pulses to the heartbeat may also provide a predictable opportunity to apply specialized waveforms at a lower repeat rate then the body may tolerate in a steady state operating mode.

It may be noted that although one aspect of the invention is described as synchronizing the electromagnetic pulse waveform change and/or substituting one or more different waveforms at about the time of the heartbeat and then returning to the original waveform, it may be that this synchronization operates at some multiple of the rate. For example, if the heartbeat is 80 beats per minute (bpm), then the synchronized changed EM pulses may be emitted at a rate of 40 changes per minute, 80 changes per minute, 160 changes per minute or at some other fractional or multiple rate that results in the change in waveform pulse characteristics being timed to match the heartbeat or pulse rate at the meridian point, or according to some other predetermined or dynamically determined rules or policies that may be customizable to the patient.

In one non-limiting example, the portable device based sensor may detect the pulse of blood passing near the point of application of the WM waves, or at a different location (such as at a meridian point) where the EM pulse is to be applied and self-synchronize with that blood flow pattern. This may be of particular benefit where the patient has some irregularity in the pulse or heartbeat. In one example, an accelerometer is used to detect the pulse generally, at the location of treatment (such as for example at the finger-tip of the hand, at a region of the foot, or at the meridian point or points). In another example, an Infrared transceiver is used to detect the pulse, such as at a finger tip location in conjunction with the palm shaped housing where the Infrared detector is positioned within the housing but has an aperture though the housing via which the infrared beam is passed. And which is located on the housing where a finger would naturally contact that aperture while holding the portable device. Where multiple portable devices are placed on the patients body, the more or less instantaneous or real-time sensing of a heartbeat, local blood flow pulse, or other sensed status may permit the EM wave pulse to be applied more nearly in synchrony than relying on a fixed frequency. For example, by way of example but not limitation, a nominal 30 Hz set of waveform pulses may be applied to a body part between heartbeats and that application rate may nominally be about once per second to synchronize with the local pulse, but if the pulse is somewhat irregular, then application frequency may track that irregular heartbeat and local pulse. If the heartbeat or pulse varies by for example $\pm$ 0.5 seconds than the EM wave pulse set may also be advanced or delayed by the same $\pm$ 0.5 seconds.

Note that since the waveform is dynamic and programmable, any of the waveform characteristics can be changed during the course of the treatment. Therefore if it is determined that efficacy is greatest if the treatment starts off with lower intensity, triangular wave and ends with a high intensity sine wave, or even some other more complex wave or sets of different waves, the inventive portable excitation terminal or device can be programmed, either directly or remotely via the system and communications interface, to do so.

It may be appreciated in light of the description provided herein that one of the special features and advantages of the present invention is the application of EM field to small localized regions of the person's body Particular beneficial effects have been determined to result from treatment at the palm surface of the hand, on the sole of the human foot, and at or along traditional acupuncture/ meridian lines, One form of device has been formed to be enclosed in a housing that is shaped to be held in the palm of the patients had during the treatment since treatment at the palm of the hand has shown very good therapeutic effects, especially relative to cardiovascular conditions.

In one non-limiting example embodiment of the computer such as a computer server and database and system that may be used with advantage to the portable excitation terminal device. The non-limiting system configuration provides a Computer or Server <--> Cellular network <--> Patient Cell Phone <--> Portable Device communication pathway. This configuration can provide several advantages, functions, and features including for example:

(1) To act as a database for storing (and retrieving) the various waveform digital encodings and maintaining a patient-to-waveform mapping correspondence.

(2) To maintain a database of the patients and the relevant details of each one (i.e., usage details, bio-feedback information, and waveform details, customizations, treatment history and results, and other information.).

(3) Provide a platform for the doctor, user, or other treatment provider or therapist to develop/edit the waveform based on such factors as treatment history, patient body and/or electrical characteristic, location(s) of treatment on the patient body (palm of the hand, sole of the foot, meridian point, region of significant blood volume flow, or other regions), and other customization factors. Factors such as skin thickness, the amount of fatty tissue underlying the treatment site, normal heartbeat rate, and/or other body factors, may also be relevant to waveform selection and usage a described in greater detail elsewhere herein.

(4) To push (or pull in response to a request) the encoded data representing the waveforms, including for example a waveform to be used for a particular treatment session, to the patient or to the portable unit to be used with the particular patient. Note that if a portable unit is to be used with different patients, then security measures or rules may be provided to assure that data sent for one patient cannot be inadvertently used for a different patient in error, and when treatment results might be combined in a statistical or other analytical manner that they patient identity is not used or exchanged between different uses.

For example, a timeout feature, or limiting the use of a treatment waveform to only one use or use during a period of time (such as for a month), or other security or safety features may be implemented. Of course, in some instances, a waveform may be somewhat generic and used as a starting point for patient customization. These generic starting waveforms may not have such stringent safety or security features.

For example, a timeout feature, or limiting the use of a treatment waveform to only one use or use during a period of time (such as for a month), or other security or safety features may be implemented. Other safety and or security features may also or alternatively be implemented in any combination, such as for example, a number of treatment sessions per day, a total treatment time per day, a maximum continuous treatment time, or other treatment restriction or permission conditions or parameters. Of course, in some instances, a waveform may be somewhat generic and used as a starting point for patient customization. These generic starting waveforms may not have such stringent safety or security features. However, even for these somewhat generic starting waveforms, restrictions may be applied.

It will also provide to the doctor who prescribes the patient treatment (or other provider), the direct interface for managing the course of treatment.

It may be appreciated in light of the description provided herein that the cellular phone channel need not be the only path where the server can download its encoded waveform files. In another manifestation it can push that information through the Internet or any network any PC so the PC can download the waveform file to the handheld device through its USB port.

It may further be appreciated that the portable excitation device may be sold to a clinic, therapist, or end user patient. Alternatively, the portable excitation unit may be rented or leased with a per use payment, a periodic payment (day, week, monthly, year, etc) or a combination of a per use and term or periodic payment.

Various subscription revenue models and business revenue generating models may be implemented, some on a subscription basis. For example, a person may obtain a cell or tissue excitation unit either for cost or as a part of the subscription and then pay for each use, total time used, customization services, analytical services, or in other ways.

Additionally or alternatively, there may be a revenue model that provides for payment to a data provider so that there is a payment due when new waveforms are downloaded or customized. This may be an additional periodic payment that provides revenues to at least offset the expenses of operating any server system. In this respect, in one alternative example, the waveforms may be uniquely identified to a particular portable excitation terminal (using some internal identifier or serial number) and optionally to a particular patient. They may also be encrypted in some manner so that they cannot be exchanged between different portable devices or different patients. This would be beneficial from both a patient safety standard and a sales and revenue standard.

Use of the portable device and/or server and database elements of the system may therefore depend on some password and identifier being input to the portable device or sent to the device via a wireless communication interface. While not required in any portable device, an input interface such as a touch screen input interface that may provide for entering numeric codes, alphanumeric codes, and/or other information may be provided. The input interface may share hardware such as a small touch screen with the display or output in some examples of the device.

Various examples of the system are described herein that may provide for communication connectivity between the portable and preferably battery powered excitation terminal or device and an external system server and database. Because of the widespread adoption and availability of cellular telephones, the ability to use such cellular telephone as a communications means between the portable excitation terminal and such server and database represents both technical advantage and cost savings because a separate communications link is not required. The advantage is particularly enhanced when the cellular phone (such as a smart phone) has a Bluetooth and/or WiFi or other local communication link so that the cellular telephone may exchange data or other information with the excitation terminal using the Bluetooth or WiFi and then the cellular telephone may communicate with the server over the longer distances using the cellular infrastructure. Using a WiFi or other network is also possible when such connectivity is provided and available.

The cellular phone may advantageously act as a bridge that will receive data through the cellular network if the patient so desires. This will provide maximum mobility to the patient when he/she moves around to different locations during the day. It also provides this communication capability at virtually no costs since it is expected that most patients will have access to a cellular phone and communication services. Furthermore, as some cellular networks and communications protocols encrypt the information communicated over the cellular infrastructure, some measure of security of sensitive patient health information is automatically provided.

Once the data is received, the cellular phone can forward the received information to the handheld unit through a localized RF channel (i.e., Bluetooth, WiFi, or other wired or wireless connection).

Optionally, the cellular phone capability may be provided within the portable device, but this is disadvantageous since it would increase the cost of the portable unit and possibly incur additional service fees.

There could be at lest two different manifestations of the cellular phone behavior: (a) an application on the cellular phone that will be responsible for receiving and forwarding the data, and (b) the portable excitation terminal or device is a mobile unit that has built-in information to trigger the cellular phone to dial out to the server.

In yet another example, the portable excitation terminal unit may optionally provide wireless communication so that it can directly send and/or receive data and waveforms without the use of the separate cellular telephone.

## Experimental Results

The waveforms and signals were at least in part identified on the basis of experimental data on actual patients and are provided without benefit of theory.

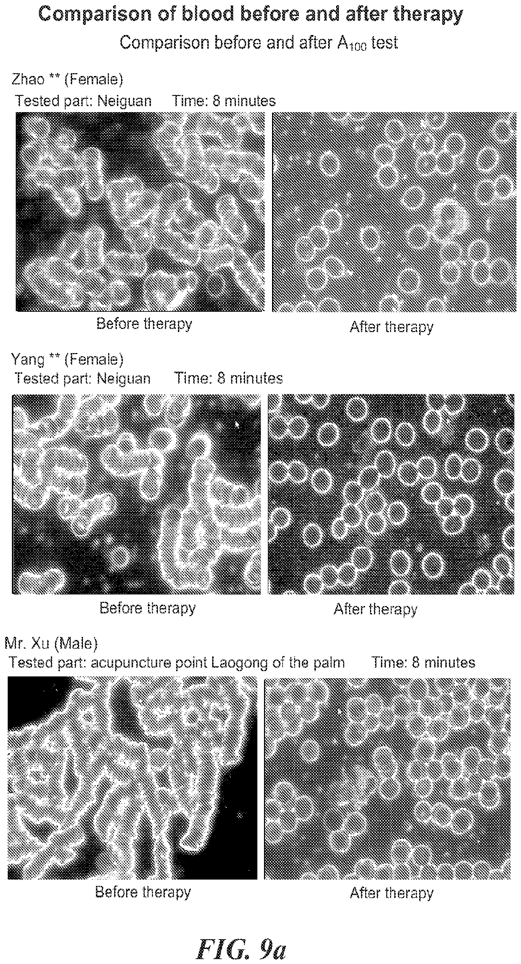

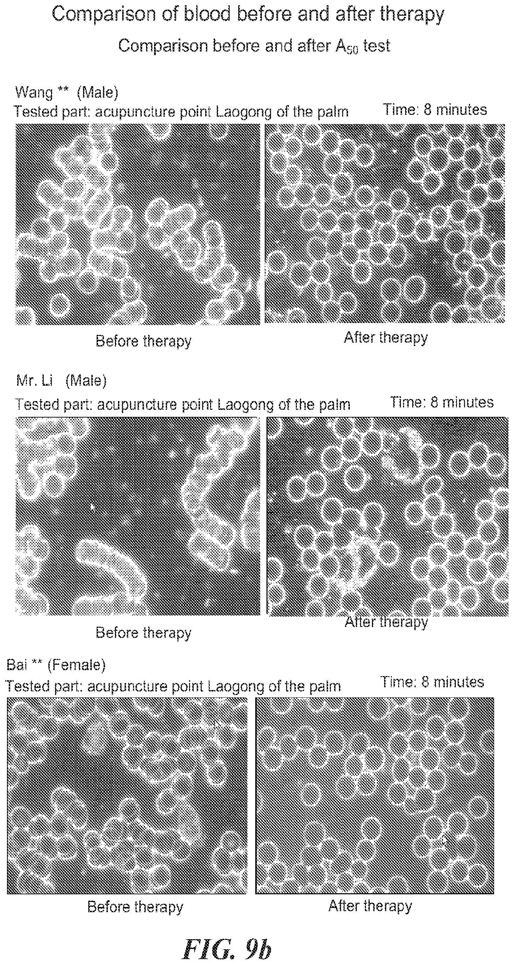

FIG. 9A and FIG. 9B provides before and after treatment comparison diagrams or photomicrographs (highly magnified blood cells) of effects on blood before and after a therapeutic scheme for six different patients according to the present invention. In FIG. 9, a comparison diagram of blood before the therapy and after the therapy using the described therapeutic device and system according to the present invention which was applied for eight minutes (8 minutes) is shown through three cases. It can be seen from the blood diagrams on the left before the therapy that many red blood cells congregate and stick together, and on the right side after eight minutes of the therapy the red blood cells disperse relatively and seldom congregate.

A condition where the red blood cells congregate and stick together are stuck together in a string form is called Rouleaux. It is a condition that signifies various issues with the health condition of the patient.

More generally, rouleau or rouleaux are generally linear stacks or clumps of red blood cells which are thought to form because of the disc-like shape of the red blood cells in vertebrate bodies such as in human patient bodies. The flat surface(s) of the discs give the red blood cells a large surface area to make contact and as a result of the contact to stick to each other or clump forming a rouleau. The rouleau are thought to form when the blood plasma protein concentration is high, and when the rouleau are present the cell or erythrocyte sedimentation rate is also increased. Rouleau and/or a high erythrocyte sedimentation rate may be an indicator of disease in the body. Conditions which cause rouleaux formation include infections, multiple myeloma, inflammatory and connective tissue disorders, and cancers, and may be associated with the development of micro vascular occlusion in diabetic retinopathy associated diabetes mellitus. Therefore, it may be appreciated that the reduction or elimination of the formation of rouleau is a significant indicator that the system, device, method, and therapeutic treatment have had a positive beneficial effect on the formation of rouleau and of an underlying sickness, malady, or disease condition.

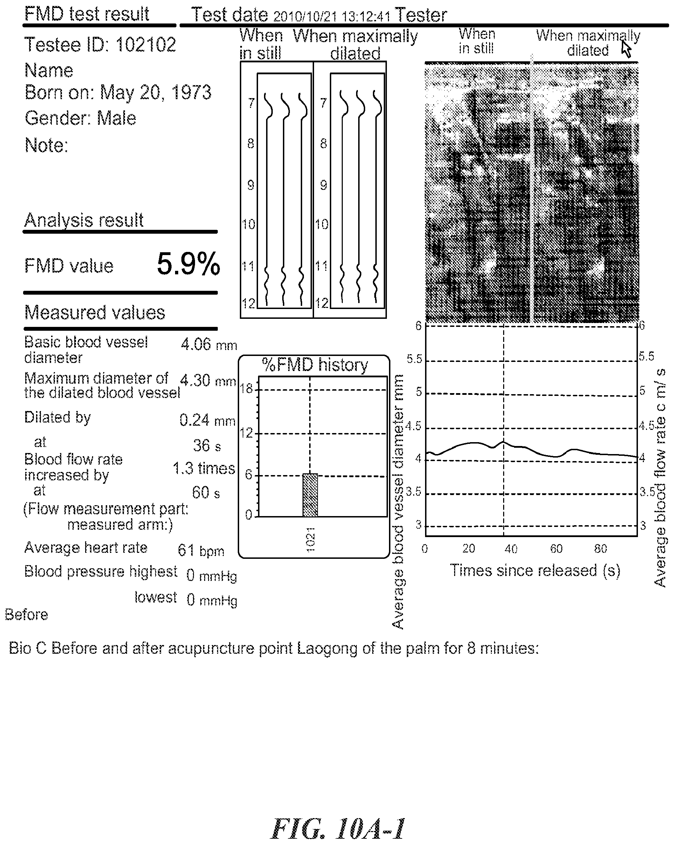

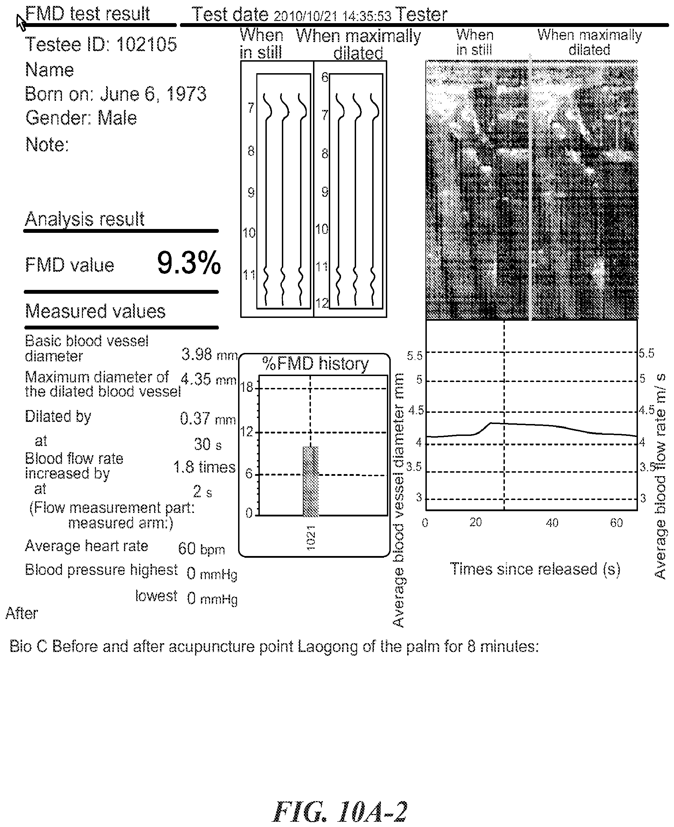

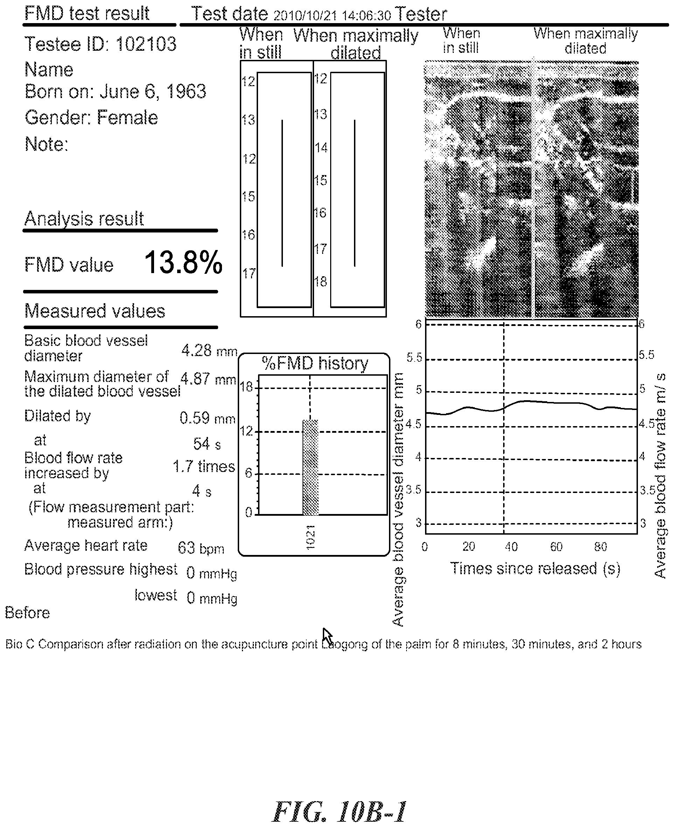

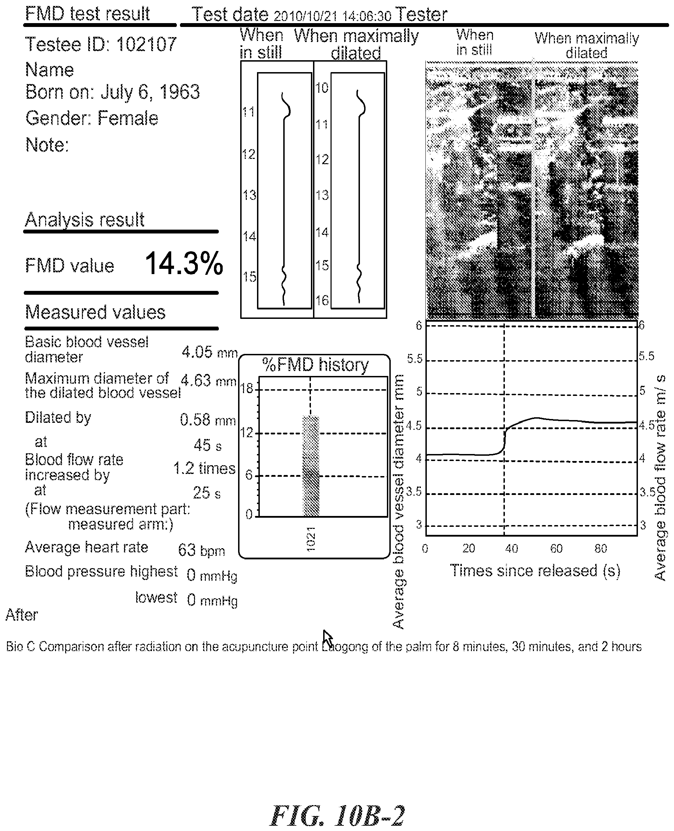

FIG. 10A and FIG. 10B are effect comparison diagrams of brachial artery flow mediated dilation (FMD) and relevant blood properties tested after a therapeutic scheme is applied on a specific treated part, an acupuncture point of the palm of the hand and named Laogong, for eight (8) minutes, thirty (30) minutes, and one-hundred twenty minutes (2 hours). It may be appreciated that the acupuncture point for a particular part of the body (such as an acupuncture point for the palm of the hand or the sole of the foot) may be located at a different region of the body than the body part itself. The brachial artery is the major blood vessel of the upper arm. As a point of reference, the pulse of the brachial artery is palpable on the anterior portion of the arm near the elbow, medial to the tendon of the biceps, and with the use of a stethoscope and sphygmomanometer or blood pressure cuff is often used as the location to measure the blood pressure.

It is known that the value of the artery flow mediated dilation or FMD indicates performance of an endothelium. It is know that the endocardial endothelium modulates the performance of the subjacent myocardium and plays an important role in regulating cardiac function. Furthermore, vascular endothelium has been shown to modify the contractile characteristics of vascular smooth muscle, and endocardial endothelium has been shown to modify the contractile characteristics of adjacent myocardium.

In FIG. 10A, for a patient, after 8 minutes the FMD increases from 5.9% to 9.3%, and blood parameters such as also improve obviously.

In FIG. 10B, for another patient, after 2 hours the FMD increases from 13.8% to 14.3%, and blood parameters also improve obviously. The term exsanguinations refers to the point or location in the body where the FMD test is applied and refers to the location where the body part or vessels are squeezed or restricted during the measurement and not to an actual bleeding.

It can be seen from data analysis according to FIG. 7 through FIG. 10 that, the therapeutic scheme according to the present invention actually achieves an obvious therapeutic benefit and technical effect, particularly when the EM waveforms of the type illustrated and described are applied to the palm of the hand or to the sole of the foot, or to both.

**Claims**

1.  A portable handheld cell or tissue excitation terminal configured for dynamic optimization of a therapeutic treatment effect, the excitation terminal comprising:

    a central processing unit (CPU);
    a human body status detection device, connected to the CPU and used to detect human body status information, the human body status detection device comprising a heartbeat detector;
    one or more electromagnetic (EM) wave generators configured to generate EM field; and
    a power device, used to supply power to operate the devices within the excitation terminal,
    wherein: each of the EM wave generators is connected to the CPU, and the CPU controls, according to a signal detected by the human body status detection device, the EM wave generator to send EM waves corresponding to a detected human body status information, and **characterised in that**
    the generated EM field (EMF) is a pulsed EMF (PEMF), and the EM wave generator generates a therapeutically effective waveform of the form:

$$I(t) = \begin{cases} 0, t < 0 \\ I_{max} \times (1 - 1/(e^{t/\tau})), 0 \le t \le t_1 \\ 0, t > t_1 \end{cases}$$

    where

    a) 300 $\mu$sec $\le \tau \le$ 0.02 sec
    b) 5 msec $\le t_1 \le$ 0.1 sec and

    - $\tau$ is a time constant of generation of the waveform generator,
    - t is any time point,
    - ($t_1$ - t) is the duration or the on-time of the pulse, and
    - $I_{max}$ is a maximum direct current (DC) following in the circuit.

2.  The cell excitation terminal according to claim 1, wherein the EM wave generator comprises a waveform multiplexer, the multiplexer operable for selecting corresponding EM waves from an available plurality of available EM signals or EM waveform signal definitions or characteristics stored in a computer, server, information appliance, smart-phone, or other external source for processing and outputting several required EM waves, wherein the available plurality of waves may be the same wave or different waves.

3.  The cell excitation terminal according to claim 1, wherein the human body status detection device further comprises a blood pressure detector.

4.  The cell excitation terminal according to claim 1, wherein the human body status detection device further comprises a motion sensor.

5.  The cell excitation terminal according to claim 1, wherein the EM wave generator comprises: a drive circuit, receiving a waveform signal from a waveform shaper generator and operable for driving an inductive circuit to generate corresponding EM waves.

6.  A therapeutic system configured for using customized electromagnetic (EM) waves varying dynamically with time for excitation, the therapeutic system comprising:
a cell or biological tissue excitation terminal according to claim 1, wherein the cell excitation terminal further comprises:

a communication device for communication between the cell excitation terminal and another device external from and outside of the cell excitation terminal; and
a computer, wherein the computer is connected to the cell excitation terminal through a wired or wireless network, and the computer receives and processes the detected human body status information sent by the cell excitation terminal, and sends an instruction or command, so that the CPU of the cell excitation terminal controls the EM wave generator to send the EM waves corresponding to the detected status or subject.

7. The therapeutic system according to claim 6, wherein the computer further comprises a data storage and a database defined therein, the database being used to store: (i) the detected human body status information, (ii) associated therapeutic information, (iii) various therapeutic waveforms, and (iv) a mapping relationship list or other data structure of patient sickness or condition and waveforms, wherein the mapping relationship list or other data structure lists therapeutic EM waves or EM wave combinations and therapeutic means corresponding to different human body status information.

8. The therapeutic system according to claim 6, wherein the EM wave generator comprises: a drive circuit, receiving a waveform signal from a waveform shaper generator and driving an EM oscillating circuit to generate corresponding EM waves.

**Patentansprüche**

1. Tragbares Zell- oder Gewebeanregungshandterminal, ausgebildet zur dynamischen Optimierung eines therapeutischen Behandlungseffekts, wobei das Anregungsterminal aufweist:

eine Zentrale Verarbeitungseinheit (CPU);
eine Vorrichtung zur Erkennung eines menschlichen Körperstatus, die mit der CPU verbunden ist und zum Erfassen von menschlichen Körperstatusinformationen verwendet wird, wobei die Vorrichtung zur Erkennung des menschlichen Körperstatus einen Herzschlagdetektor aufweist;
einen oder mehrere elektromagnetische (EM) Wellengeneratoren, ausgebildet zum Erzeugen eines EM-Feldes; und
ein Stromversorgungsgerät, das zur Stromversorgung für den Betrieb der Geräte innerhalb des Anregungsterminals verwendet wird,
wobei jeder der EM-Wellengeneratoren mit der CPU verbunden ist und die CPU gemäß einem von der Vorrichtung zur Erkennung des menschlichen Körperstatus erfassten Signal den EM-Wellengenerator steuert, um EM-Wellen zu senden, die einer erfassten menschlichen Körperstatusinformation entsprechen,
und **dadurch gekennzeichnet, dass**
das erzeugte EM-Feld (EMF) ein gepulstes EMF (PEMF) ist,
und dass der EM-Wellengenerator eine therapeutisch wirksame Wellenform erzeugt der Form:

$$I(t) = \begin{cases} 0, t < 0 \\ I_{max} \times \left(1 - \dfrac{1}{e^{\frac{t}{\tau}}}\right), 0 \le t \le t_1 \\ 0, t > t_1 \end{cases}$$

wobei

a) 300 μs $\le \tau \le$ 0,02 s
b) 5 ms $\le t_1 \le$ 0,1 s
und

- $\tau$ eine Erzeugungs-Zeitkonstante des Wellenformgenerators ist,
- t ein beliebiger Zeitpunkt ist,
- (ti - t) die Dauer oder die Einschaltdauer des Pulses ist, und

- $I_{max}$ ein maximaler Gleichstrom (DC) ist, der in der Schaltung folgt.

2. Zellanregungsterminal nach Anspruch 1, wobei der EM-Wellengenerator einen Wellenform-Multiplexer aufweist, wobei der Multiplexer ausgebildet ist zum Auswählen entsprechender EM-Wellen aus einer verfügbaren Mehrzahl von verfügbaren EM-Signalen oder EM-Wellenformsignaldefinitionen oder Charakteristiken, gespeichert in einem Computer, Server, Informationsgerät, Smartphone oder einer anderen externen Quelle zum Verarbeiten und Ausgeben mehrerer erforderlicher EM-Wellen, wobei die verfügbare Mehrzahl von Wellen dieselbe Welle oder verschiedene Wellen sein kann.

3. Zellanregungsterminal nach Anspruch 1, wobei die Vorrichtung zur Erkennung des menschlichen Körperstatus zudem einen Blutdruckdetektor aufweist.

4. Zellanregungsterminal nach Anspruch 1, wobei die Vorrichtung zur Erkennung des menschlichen Körperstatus zudem einen Bewegungssensor aufweist.

5. Zellanregungsterminal nach Anspruch 1, wobei der EM-Wellengenerator eine Ansteuerschaltung aufweist, die ein Wellenformsignal von einem Wellenform-Formungsgenerator empfängt und zum Ansteuern einer induktiven Schaltung zum Erzeugen entsprechender EM-Wellen betreibbar ist.

6. Therapeutisches System, ausgebildet zum Verwenden angepasster elektromagnetischer (EM) Wellen, die sich dynamisch mit der Zeit ändern, für eine Anregung, wobei das therapeutische System aufweist:
ein Zell- oder biologisches Gewebeanregungsterminal nach Anspruch 1, wobei das Zellanregungsterminal ferner aufweist:

   eine Kommunikationsvorrichtung zur Kommunikation zwischen dem Zellenanregungsterminal und einer anderen Vorrichtung extern und außerhalb des Zellenanregungsterminals; und
   einen Computer, wobei der Computer über ein drahtgebundenes oder drahtloses Netzwerk mit dem Zellenanregungsterminal verbunden ist und der Computer die von dem Zellanregungsterminal gesendeten erfassten menschlichen Körperstatusinformationen empfängt und verarbeitet und eine Anweisung oder einen Befehl sendet, so dass die CPU des Zellanregungsterminals den EM-Wellengenerator steuert, die dem erfassten Status oder Subjekt entsprechenden EM-Wellen zu senden.

7. Therapeutisches System nach Anspruch 6, wobei der Computer ferner einen Datenspeicher und eine darin definierte Datenbank aufweist, wobei die Datenbank verwendet wird zum Speichern (i) der erfassten menschlichen Körperstatusinformation, (ii) damit verbundener therapeutischer Informationen, (iii) verschiedener therapeutischer Wellenformen und (iv) eine Zuordnungsbeziehungsliste oder eine andere Datenstruktur einer Krankheit oder eines Zustands des Patienten und Wellenformen, wobei die Zuordnungsbeziehungsliste oder die andere Datenstruktur therapeutische EM-Wellen oder EM-Wellenkombinationen und therapeutische Mittel auflistet, die unterschiedlichen menschlichen Körperstatusinformationen entsprechen.

8. Therapeutisches System nach Anspruch 6, wobei der EM-Wellengenerator eine Ansteuerschaltung aufweist, die ein Wellenformsignal von einem Wellenform-Formungsgenerator empfängt und eine EM-Oszillationsschaltung ansteuert, entsprechende EM-Wellen zu erzeugen.

**Revendications**

1. Terminal portable à main d'excitation de cellules ou de tissu configuré pour l'optimisation dynamique d'un effet de traitement thérapeutique, le terminal d'excitation comprenant:

   une unité centrale de traitement (CPU);
   un dispositif de détection d'état du corps humain, connecté à l'unité centrale et utilisé pour détecter des informations d'état du corps humain, le dispositif de détection d'état du corps humain comprenant un détecteur de pulsations cardiaques;
   un ou plusieurs générateurs d'ondes électromagnétiques (EM) configurés pour générer un champ EM; et
   un dispositif d'alimentation électrique, utilisé pour l'alimentation électrique pour l'opération des dispositifs dans le terminal d'excitation,
   dans lequel chacun des générateurs d'ondes EM est connecté à la CPU, et la CPU commande, selon un signal détecté par le dispositif de détection d'état du corps humain, le générateur d'ondes EM pour envoyer des ondes EM correspondant à une information d'état du corps humain détectée,
et **caractérisé en ce que**
le champ EM généré (EMF) est un EMF pulsé (PEMF),
et le générateur d'ondes EM génère une forme d'onde thérapeutiquement efficace de la forme:

$$I(t) = \begin{cases} 0, t < 0 \\ I_{max} \times \left(1 - \dfrac{1}{e^{\frac{t}{\tau}}}\right), 0 \le t \le t_1 \\ 0, t > t_1 \end{cases}$$

où

a) 300 $\mu$s $\le \tau \le$ 0,02 s
b) 5 ms $\le t_1 \le$ 0,1 s
et

- $\tau$ est une constante de temps de génération du générateur de forme d'onde,
- t est un moment quelconque,
- (ti - t) est la durée ou la durée d'activation de l'impulsion, et
- $I_{max}$ est un maximum de courant continu (CC) suivant dans le circuit.

2. Terminal d'excitation de cellules selon la revendication 1,
dans lequel le générateur d'ondes EM comprend un multiplexeur de formes d'ondes, le multiplexeur fonctionnant pour sélectionner des ondes EM correspondantes parmi une pluralité disponible de signaux EM disponibles ou de définitions ou de caractéristiques de signaux de formes d'ondes EM stockées dans un ordinateur, serveur, appareil d'information, smartphone ou autre source externe pour le traitement et la sortie de plusieurs ondes EM requises, dans lequel la pluralité disponible d'ondes peut être la même onde ou des ondes différentes.

3. Terminal d'excitation de cellules selon la revendication 1, dans lequel le dispositif de détection d'état du corps humain comprend en outre un détecteur de tension artérielle.

4. Terminal d'excitation de cellules selon la revendication 1, dans lequel le dispositif de détection d'état du corps humain comprend en outre un capteur de mouvement.

5. Terminal d'excitation de cellules selon la revendication 1, dans lequel le générateur d'ondes EM comprend un circuit de commande, recevant un signal de forme d'onde d'un générateur de mise en forme de forme d'onde et pouvant être actionné pour commander un circuit inductif pour générer des ondes EM correspondantes.

6. Système thérapeutique configuré pour utiliser des ondes électromagnétiques (EM) personnalisées variant dynamiquement avec le temps pour l'excitation, le système thérapeutique comprenant:
un terminal d'excitation de cellules ou de tissu biologique selon la revendication 1, dans lequel le terminal d'excitation de cellules comprend en outre:

un dispositif de communication pour la communication entre le terminal d'excitation de cellules et un autre dispositif externe à partir et à l'extérieur du terminal d'excitation de cellules; et
un ordinateur, dans lequel l'ordinateur est connecté au terminal d'excitation de cellules via un réseau filaire ou sans fil, et l'ordinateur reçoit et traite les informations d'état du corps humain détectées envoyées par le terminal d'excitation de cellules, et envoie une instruction ou une commande, de sorte que la CPU du terminal d'excitation de cellules commande le générateur d'ondes EM pour envoyer les ondes EM correspondant à l'état ou au sujet détecté.

7. Système thérapeutique selon la revendication 6, dans lequel l'ordinateur comprend en outre un stockage de données et une base de données définie dans celui-ci, la base de données étant utilisée pour stocker: (i) les informations d'état du corps humain détectées, (ii) des informations thérapeutiques associées, (iii) diverses formes d'ondes thérapeutiques, et (iv) une liste de relations de classement ou une autre structure de données de la maladie ou de l'état du patient et des formes d'onde, la liste de relations de classement ou l'autre structure de données listant des ondes EM thérapeutiques ou des combinaisons d'ondes EM et des moyens thérapeutiques correspondant à différentes informations d'état du corps humain.

8. Système thérapeutique selon la revendication 6, dans lequel le générateur d'onde EM comprend un circuit de commande, recevant un signal de forme d'onde d'un générateur de mise en forme de forme d'onde et commandant un circuit oscillant EM pour générer des ondes EM correspondantes.

**FIG. 1**

Portable therapeutic device — 5

Public wireless network — 3

Server 1
- Database 11
- Model optimization module 12
- Communication function module 13

*FIG. 2*

**FIG. 3**

EP 2 624 910 B1

Detect physical status of a human body ～ 402

Compare associated therapeutic information of the human body ～ 403

Search a mapping relationship list of patient sickness and waveforms for suitable waveforms ～ 404

Customize a therapeutic waveform (set) and a therapeutic manner suitable for the patient ～ 405

Generate corresponding EM waves to perform radiation on the patient ～ 406

Measure a therapeutic effect and optimize the mapping relationship list of patient sickness and waveforms ～ 407

400

*FIG. 4*

*500* ↘

Listing: therapy on a patient (Mr. Wang) — *501-1*

Perform listing for patients of a period of time — *501-2*

Sorting (grouping) — *502*

Comparison: perform comparison according to effects — *503*

Update: Replace original bad data

## FIG. 5

mA

I

*706*

*707*

*705*

0

time — ne

## FIG. 7

| Sickness ~ 601 | Therapeutic waveform ~ 602 | Therapeutic means (manner) ~ 603 |
|---|---|---|
| **I**<br>Heart Disease<br>~ 611 { Heart Disease A1 | Waveform P1 | Time T1 |
| Heart Disease A2 | Waveform P1+waveform P3 | Time T1+cycle S1 |
| **II**<br>High Blood Pressure<br>~ 612 { Hypertension B1 | Waveform P4 | Time T3 |
| Hypertension B2 | Waveform P5+waveform P6 | Time T4+cycle S2 |
| Hypertension B3 | Waveform P6+waveform P7+ waveform P8 | Time T4+cycle S3 |

*FIG. 6*

EP 2 624 910 B1

| Tested person 801 | Symptoms 802 | Testing scheme 803 | Time and times for testing 804 | Assessment of the test 805 | Scheme/ therapeutic effect feasibility 806 |
|---|---|---|---|---|---|
| Yao** 811 | Headache caused by catching a head cold | Bio A aching part radiation | The test time span is 1.5 hr, and includes 6 times (treatment sessions) | Symptoms disappeared | Obviously effective |
| Bai** 812 | Lumbago | $A_{100}$ local radiation | The test time span is 1 day, and includes 1 time | Symptoms alleviated | Obviously effective |
| Chen** 813 | Insomnia | $A_{50}$ radiation on palm | The test time span is 30 days, and includes 7 times | Symptoms obviously alleviated | Obviously effective |
| Wang** 814 | Dried blood, and empty felt at stomachs | Biology energy level 5 and $A_{50}$ local radiation | The test time span is 19 days, and includes 33 times at the biologic energy level 5 and 15 times of $A_{50}$ | Emptiness remained unchanged at the biologic energy level 5, and the emptiness disappear at $A_{50}$ | Ineffective at biologic energy level 5, and obviously effective at $A_{50}$ |
| Bai** 815 | lumbago (lower back pain) for days | $A_{100}$ local aching point radiation | Totally once | Aching was alleviated after one hour, and aching did not occur next day | Obviously effective |
| Wang** 816 | A part of the left instep neat the ankle has ached for two days | $A_{50}$ local aching point radiation | The test time span in 6 days, and included 24 times | Aching was alleviated on first day, discomfort was not felt for consecutive days, but curing was not achieved, aching disappeared 6th day | Obviously effective |
| Bai** 817 | Palpitation, a too high heart rate | $A_{100}$ radiation on acupuncture point Laogong of palm | The test time span is 1 days, and includes 2 times | Symptoms disappeared | Obviously effective |
| Ren** 818 | Bloodshot fundus of the eye | Bio D local radiation | Totally two times, and 8 minutes for each time | Bloodshot fundus is alleviated for first time, and symptoms disappeared after two times | Cured |
| Ren** 819 | Narrow right carotid | Bio C radiation on right carotid and left hand radial artery | The test time span is 46 days, and includes 72 times at right carotid and 35 times at the left hand radial artery | The right carotid grows from 6.3mm to 7.2 mm, and no other discomfort was felt | Obviously effective |

## FIG. 8

# Comparison of blood before and after therapy

Comparison before and after $A_{100}$ test

Zhao ** (Female)
Tested part: Neiguan     Time: 8 minutes

Before therapy                    After therapy

Yang ** (Female)
Tested part: Neiguan     Time: 8 minutes

Before therapy                    After therapy

Mr. Xu (Male)
Tested part: acupuncture point Laogong of the palm     Time: 8 minutes

Before therapy                    After therapy

*FIG. 9a*

Comparison of blood before and after therapy

Comparison before and after A50 test

Wang ** (Male)
Tested part: acupuncture point Laogong of the palm     Time: 8 minutes

Before therapy                                    After therapy

Mr. Li   (Male)
Tested part: acupuncture point Laogong of the palm     Time: 8 minutes

Before therapy                                    After therapy

Bai ** (Female)
Tested part: acupuncture point Laogong of the palm     Time: 8 minutes

Before therapy                                    After therapy

*FIG. 9b*

FMD test result     Test date 2010/10/21 13:12:41 Tester

Testee ID: 102102
Name
Born on: May 20, 1973
Gender: Male
Note:

Analysis result

FMD value     **5.9%**

Measured values

| | |
|---|---|
| Basic blood vessel diameter | 4.06 mm |
| Maximum diameter of the dilated blood vessel | 4.30 mm |
| Dilated by | 0.24 mm |
| at | 36 s |
| Blood flow rate increased by | 1.3 times |
| at | 60 s |
| (Flow measurement part: measured arm:) | |
| Average heart rate | 61 bpm |
| Blood pressure highest | 0 mmHg |
| lowest | 0 mmHg |
| Before | |

When in still   When maximally dilated

%FMD history

When in still   When maximally dilated

Average blood vessel diameter mm

Average blood flow rate c m/ s

Times since released (s)

Bio C Before and after acupuncture point Laogong of the palm for 8 minutes:

*FIG. 10A-1*

FMD test result     Test date 2010/10/21 14:35:53 Tester

Testee ID: 102105
Name
Born on: June 6, 1973
Gender: Male
Note:

When in still    When maximally dilated       When in still      When maximally dilated

## Analysis result

FMD value    **9.3%**

## Measured values

| | |
|---|---|
| Basic blood vessel diameter | 3.98 mm |
| Maximum diameter of the dilated blood vessel | 4.35 mm |
| Dilated by | 0.37 mm |
| at | 30 s |
| Blood flow rate increased by | 1.8 times |
| at | 2 s |
| (Flow measurement part: measured arm:) | |
| Average heart rate | 60 bpm |
| Blood pressure highest | 0 mmHg |
| lowest | 0 mmHg |

%FMD history

Average blood vessel diameter mm

Times since released (s)

Average blood flow rate m/ s

After

Bio C Before and after acupuncture point Laogong of the palm for 8 minutes:

*FIG. 10A-2*

FMD test result    Test date 2010/10/21 14:06:30 Tester

Testee ID: 102103
Name
Born on: June 6, 1963
Gender: Female
Note:

| When in still | When maximally dilated |
|---|---|
| 12 | 12 |
| 13 | 13 |
| 12 | 14 |
| 15 | 15 |
| 16 | 16 |
| 17 | 17 |
| | 18 |

Analysis result

FMD value **13.8%**

Measured values

Basic blood vessel diameter   4.28 mm

Maximum diameter of the dilated blood vessel   4.87 mm

Dilated by   0.59 mm
at   54 s
Blood flow rate increased by   1.7 times
at   4 s
(Flow measurement part: measured arm:)

Average heart rate   63 bpm
Blood pressure highest   0 mmHg
     lowest   0 mmHg
Before

%FMD history

When in still    When maximally dilated

Times since released (s)

Average blood vessel diameter mm

Average blood flow rate m/s

Bio C Comparison after radiation on the acupuncture point Laogong of the palm for 8 minutes, 30 minutes, and 2 hours

*FIG. 10B-1*

FMD test result     Test date 2010/10/21 14:06:30 Tester

Testee ID: 102107
Name
Born on: July 6, 1963
Gender: Female
Note:

| When in still | When maximally dilated | When in still | When maximally dilated |

Analysis result

FMD value **14.3%**

Measured values

Basic blood vessel diameter    4.05 mm

Maximum diameter of the dilated blood vessel    4.63 mm

Dilated by    0.58 mm
   at    45 s
Blood flow rate increased by    1.2 times
   at    25 s
(Flow measurement part: measured arm:)
Average heart rate    63 bpm
Blood pressure highest    0 mmHg
     lowest    0 mmHg
After

%FMD history

Times since released (s)

Average blood vessel diameter mm

Average blood flow rate m/s

Bio C Comparison after radiation on the acupuncture point Laogong of the palm for 8 minutes, 30 minutes, and 2 hours

*FIG. 10B-2*

**FIG. 11**

*FIG. 12A*

*FIG. 12B*

EP 2 624 910 B1

1157

**FIG. 12C**

1162

LCD

U5
LCD

| Pin | Name |
|---|---|
| 1 | NC |
| 2 | Vss |
| 3 | Vcc |
| 4 | VCOMH |
| 5 | IREF |
| 6 | D7 |
| 7 | D6 |
| 8 | D5 |
| 9 | D4 |
| 10 | D3 |
| 11 | D2 |
| 12 | D1 |
| 13 | D0 |
| 14 | RD |
| 15 | WR |
| 16 | D/C |
| 17 | RES |
| 18 | CS |
| 19 | BS2 |
| 20 | BS1 |
| 21 | Vdd-IO |
| 22 | Vdd |
| 23 | Vss |
| 24 | Vcc |
| 25 | NC |

Va  Va

Va  Vc

R45

R42

R41

R44

SDA
SCL

SS

R46

C13

C15 C14   C16   R50

*FIG. 12D*

*FIG. 12E*

EP 2 624 910 B1

*FIG. 12F*

EP 2 624 910 B1

EP 2 624 910 B1

1160

## Vc Switching Supply

L4

Vsys

U10

SW

6 Vin    Vout 5

3 EN     Vfb 4

GND

2

C31

R64

R66

Vc

C29

**FIG. 12G**

Va LDO

FIG. 12H

EP 2 624 910 B1

*FIG. 13A*

*FIG. 13B*

*FIG. 13C*

*FIG. 13D*

**REFERENCES CITED IN THE DESCRIPTION**

**Patent documents cited in the description**

- WO 2005084753 A
- US 20100004551 A
- WO 2010025114 A
- WO 2010006175 A
- US 7801585 B
- US 5458142 A